# EUROPÄISCHE PATENTSCHRIFT

(11) **EP 0 608 739 B1**
(45) Veröffentlichungstag und Bekanntmachung des Hinweises auf die Patenterteilung: **04.04.2001**
(21) Anmeldenummer: 94100566.2
(22) Anmeldetag: 17.01.1994
(51) Int. Cl.: C07C 233/60, C07C 329/06, C07C 271/48, A01N 37/24, A01N 47/22

(54) **Cycloalkylcarbonsäureanilide**
Cycloalkyl carboxylic acid anilides
Anilides d'acides cycloalkylcarboxyliques

(30) Priorität: 28.01.1993 DE 4302305
(43) Veröffentlichungstag der Anmeldung: 03.08.1994
(73) Patentinhaber: BAYER AG, 51368 Leverkusen (DE)
(72) Erfinder: Krüger, Bernd-Wieland, Dr., D-51467 Bergisch Gladbach (DE); Sasse, Klaus, Dr., D-51467 Bergisch Gladbach (DE); Schenke, Thomas, Dr., D-51469 Bergisch Gladbach (DE); Negele, Michael, Dr., D-42697 Solingen (DE); Dehne, Heinz-Wilhelm, Dr., D-40789 Monheim (DE)

(56) Entgegenhaltungen:
- EP-A- 0 293 718
- EP-A- 0 416 359
- EP-A- 0 416 365
- DE-A- 4 120 904
- US-A- 4 166 735

## Beschreibung

Die vorliegende Erfindung betrifft neue Cycloalkylcarbonsäureanilide, ein Verfahren zu deren Herstellung und deren Verwendung zur Bekämpfung von Schädlingen.

Es ist bereits bekannt geworden daß zahlreiche Cycloalkylcarbonsäureanilide fungizide Eigenschaften besitzen (vgl. EP-OS 0 416 365 und EP-OS 0 416 359). So läßt sich z.B. 4-Acetyloxy-2,3-dichlor-(1-methyl-cyclohexancarbonsäure)-anilid zur Bekämpfung von Pilzen verwenden. Die Wirkung dieses Stoffes ist gut, läßt aber bei niedrigen Aufwandmengen in manchen Fällen zu wünschen übrig.

Aus dem Patent US-4,166,735 sind Cycloalkylcarbonsäureanilide mit herbiziden Eigenschaften erkannt.

Es wurden nun neue Cycloalkylcarbonsäureanilide der Formel 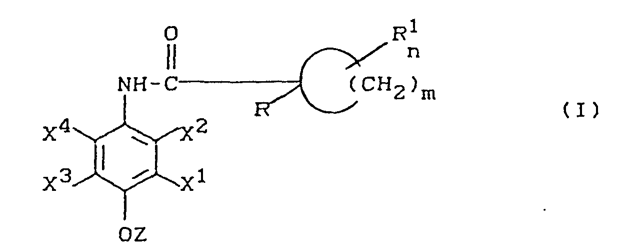 in welcher
- R: für Halogen oder Halogenalkyl steht,
- R¹: für Halogen, Alkyl oder Halogenalkyl steht,
- m: für ganze Zahlen von 2 bis 7 steht,
- n: für die Zahlen 0, 1, 2 oder 3 steht,
- X¹, X², X³ und X⁴: unabhängig voneinander für Wasserstoff, Halogen, Alkyl, Halogenalkyl, Alkoxy, Halogenalkoxy, Alkylthio oder Halogenalkylthio stehen und
- Z: für Wasserstoff oder die Reste der Formeln 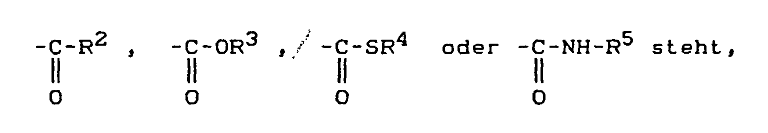 worin
R² für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht,
R³ für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht,
R⁴ für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht und
R⁵ für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht,
gefunden.

Weiterhin wurde gefunden, daß man Cycloalkylcarbonsäureanilide der Formel (I) erhält, wenn man Aminophenole der Formel 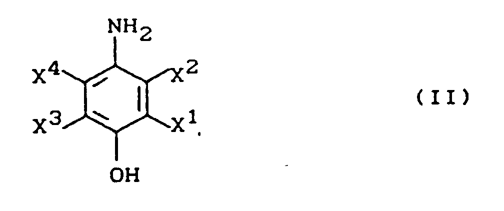 in welcher
X¹, X², X³ und X⁴ die oben angegebenen Bedeutungen haben,
mit Cycloalkylcarbonsäure-Derivaten der Formel 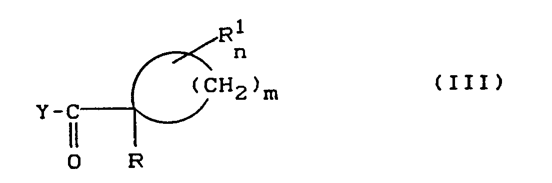 in welcher
R, R¹, m und n die oben angegebenen Bedeutungen haben und
Y für eine Leaving-Group steht,
gegebenenfalls in Gegenwart eines Säurebindemittels und gegebenenfalls in Gegenwart eines Verdünnungsmittels umsetzt und gegebenenfalls die dabei entstehenden Cycloalkylcarbonsäureanilide der Formel 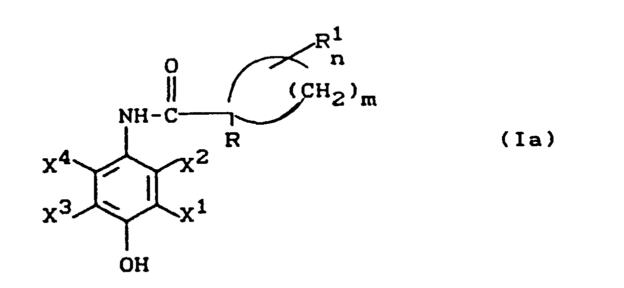 in welcher
R, R¹, m, n, X¹, X², X³ und X⁴ die oben angegebenen Bedeutungen haben,
entweder
a) mit Carbonsäurehalogeniden der Formel 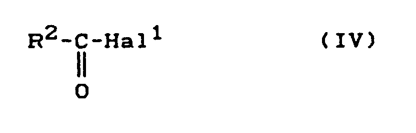 in welcher
   R² die oben angegebene Bedeutung hat und Hal¹ für Halogen steht,
   gegebenenfalls in Gegenwart eines Säurebindemittels und gegebenenfalls in Gegenwart eines Verdünnungsmittels umsetzt,
   oder
b) mit Halogenameisensäureestern der Formel 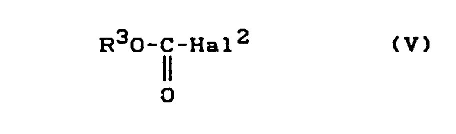 in welcher
   R³ die oben angegebene Bedeutung hat und Hal² für Halogen steht,
   gegebenenfalls in Gegenwart eines Säurebindemittels und gegebenenfalls in Gegenwart eines Verdünnungsmittels umsetzt,
   oder
c) mit Halogenameisensäure-thioestern der Formel 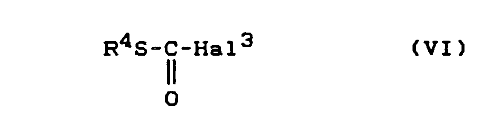 in welcher
   R⁴ die oben angegebene Bedeutung hat und Hal³ für Halogen steht,
   gegebenenfalls in Gegenwart eines Säurebindemittels und gegebenenfalls in Gegenwart eines Verdünnungsmittels umsetzt,
   oder
d) mit Isocyanaten der Formel

   R⁵-NCO (VII)

   in welcher
   R⁵ die oben angegebene Bedeutung hat,
   gegebenenfalls in Gegenwart eines Verdünnungsmittels und gegebenenfalls in Gegenwart eines Katalysators umsetzt.

Schließlich wurde nun gefunden, daß die erfindungsgemäßen Cycloalkylcarbonsäureanilide der Formel (I) sehr gut zur Bekämpfung von Schädlingen, insbesondere von Pilzen geeignet sind.

Überraschenderweise besitzen die erfindungsgemäßen Cycloalkylcarbonsäureanilide eine bessere fungizide Wirksamkeit als das 4-Acetyloxy-2,3-dichlor-(1-methylcyclohexancarbonsäure)-anilid, welches ein konstitutionell ähnlicher, vorbekannter Wirkstoff gleicher Wirkungsrichtung ist.

Die erfindungsgemäßen Cycloalkylcarbonsäureanilide sind durch die Formel (I) allgemein bekannt.
- R: steht vorzugsweise für Fluor, Chlor, Brom, Iod oder Halogenalkyl mit 1 bis 3 Kohlenstoffatomen und 1 bis 5 gleichen oder verschiedenen Halogenatomen.
- R¹: steht vorzugsweise für Fluor, Chlor, Brom, geradkettiges oder verzweigtes Alkyl mit 1 bis 4 Kohlenstoffatomen oder für Halogenalkyl mit 1 bis 3 Kohlenstoffatomen und 1 bis 5 gleichen oder verschiedenen Halogenatomen.
- m: steht vorzugsweise für ganze Zahlen von 2 bis 6.
- n: steht auch vorzugsweise für die Zahlen 0, 1, 2 und 3. Wenn n für 2 oder 3 steht, können die Reste R¹ gleich oder verschieden sein.
- X¹: steht vorzugsweise für Wasserstoff, Fluor, Chlor, Brom, Iod, geradkettiges oder verzweigtes Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen oder für Halogenalkylthio mit 1 bis 3 Kohlenstoffatomen und 1 bis 5 gleichen oder verschiedenen Halogenatomen.
- X²: steht vorzugsweise für Wasserstoff, Fluor, Chlor, Brom, Iod, geradkettiges oder verzweigtes Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen oder für Halogenalkylthio mit 1 bis 3 Kohlenstoffatomen und 1 bis 5 gleichen oder verschiedenen Halogenatomen.
- X³: steht vorzugsweise für Wasserstoff, Fluor, Chlor, Brom, Iod, geradkettiges oder verzweigtes Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen oder für Halogenalkylthio mit 1 bis 3 Kohlenstoffatomen und 1 bis 5 gleichen oder verschiedenen Halogenatomen.
- X⁴: steht vorzugsweise für Wasserstoff, geradkettiges oder verzweigtes Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen oder für Halogenalkylthio mit 1 bis 3 Kohlenstoffatomen und 1 bis 5 gleichen oder verschiedenen Halogenatomen.
- Z: steht auch vorzugsweise für Wasserstoff oder die Reste der Formeln 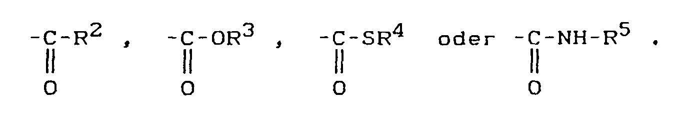
- R²: steht vorzugsweise für geradkettiges oder verzweigtes Alkyl mit 1 bis 6 Kohlenstoffatomen, Halogenalkyl mit 1 bis 6 Kohlenstoffatomen und 1 bis 7 gleichen oder verschiedenen Halogenatomen, Alkenyl mit 2 bis 6 Kohlenstoffatomen, Halogenalkenyl mit 2 bis 6 Kohlenstoffatomen und 1 bis 7 gleichen oder verschiedenen Halogenatomen, Alkoxyalkyl mit 1 bis 6 Kohlenstoffatomen im Alkylteil und 1 bis 6 Kohlenstoffatomen im Alkoxyteil, Alkoxyalkoxyalkyl mit 1 bis 6 Kohlenstoffatomen im Alkylteil und 1 bis 6 Kohlenstoffatomen in jedem Alkoxyteil, Alkylthioalkyl mit 1 bis 6 Kohlenstoffatomen im Alkylteil und 1 bis 6 Kohlenstoffatomen im Alkylthioteil, Alkylcarbonyloxyalkyl mit 1 bis 6 Kohlenstoffatomen im Alkylteil und 1 bis 6 Kohlenstoffatomen im Oxyalkylteil, Alkenylcarbonyloxyalkyl mit 2 bis 6 Kohlenstoffatomen im Alkenylteil und 1 bis 6 Kohlenstoffatomen im Oxyalkylteil oder für gegebenenfalls einfach bis dreifach, gleichartig oder verschieden durch Halogen und/oder Alkyl mit 1 bis 4 Kohlenstoffatomen substituiertes Cycloalkyl mit 3 bis 8 Kohlenstoffatomen, oder für Phenyl, das einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen, oder
für Phenylalkyl mit 1 bis 4 Kohlenstoffatomen im Alkylteil, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen, oder für Phenoxyalkyl mit 1 bis 4 Kohlenstoffatomen im Alkylteil, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 3 gleichen oder verschiedenen Halogenatomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen.
- R³: steht vorzugsweise für geradkettiges oder verzweigtes Alkyl mit 1 bis 6 Kohlenstoffatomen, Halogenalkyl mit 1 bis 6 Kohlenstoffatomen und 1 bis 7 gleichen oder verschiedenen Halogenatomen, Alkenyl mit 2 bis 6 Kohlenstoffatomen, Halogenalkenyl mit 2 bis 6 Kohlenstoffatomen und 1 bis 7 gleichen oder verschiedenen Halogenatomen, Alkoxyalkyl mit 1 bis 6 Kohlenstoffatomen im Alkylteil und 1 bis 6 Kohlenstoffatomen im Alkoxyteil, Alkoxyalkoxyalkyl mit 1 bis 6 Kohlenstoffatomen im Alkylteil und 1 bis 6 Kohlenstoffatomen in jedem Alkoxyteil, Alkylthioalkyl mit 1 bis 6 Kohlenstoffatomen im Alkylteil und 1 bis 6 Kohlenstoffatomen im Alkylthioteil, Alkylcarbonyloxyalkyl mit 1 bis 6 Kohlenstoffatomen im Alkylteil und 1 bis 6 Kohlenstoffatomen im Oxyalkylteil, Alkenylcarbonyloxyalkyl mit 2 bis 6 Kohlenstoffatomen im Alkenylteil und 1 bis 6 Kohlenstoffatomen im Oxyalkylteil oder für gegebenenfalls einfach bis dreifach, gleichartig oder verschieden durch Halogen und/oder Alkyl mit 1 bis 4 Kohlenstoffatomen substituiertes Cycloalkyl mit 3 bis 8 Kohlenstoffatomen, oder für Phenyl, das einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen, oder
für Phenylalkyl mit 1 bis 4 Kohlenstoffatomen im Alkylteil, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen, oder
für Phenoxyalkyl mit 1 bis 4 Kohlenstoffatomen im Alkylteil, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 3 gleichen oder verschiedenen Halogenatomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen.
- R⁴: steht vorzugsweise für geradkettiges oder verzweigtes Alkyl mit 1 bis 6 Kohlenstoffatomen, Halogenalkyl mit 1 bis 6 Kohlenstoffatomen und 1 bis 7 gleichen oder verschiedenen Halogenatomen, Alkenyl mit 2 bis 6 Kohlenstoffatomen, Halogenalkenyl mit 2 bis 6 Kohlenstoffatomen und 1 bis 7 gleichen oder verschiedenen Halogenatomen, Alkoxyalkyl mit 1 bis 6 Kohlenstoffatomen im Alkylteil und 1 bis 6 Kohlenstoffatomen im Alkoxyteil, Alkoxyalkoxyalkyl mit 1 bis 6 Kohlenstoffatomen im Alkylteil und 1 bis 6 Kohlenstoffatomen in jedem Alkoxyteil, Alkylthioalkyl mit 1 bis 6 Kohlenstoffatomen im Alkylteil und 1 bis 6 Kohlenstoffatomen im Alkylthioteil, Alkylcarbonyloxyalkyl mit 1 bis 6 Kohlenstoffatomen im Alkylteil und 1 bis 6 Kohlenstoffatomen im Oxyalkylteil, Alkenylcarbonyloxyalkyl mit 2 bis 6 Kohlenstoffatomen im Alkenylteil und 1 bis 6 Kohlenstoffatomen im Oxyalkylteil oder für gegebenenfalls einfach bis dreifach, gleichartig oder verschieden durch Halogen und/oder Alkyl mit 1 bis 4 Kohlenstoffatomen substituiertes Cycloalkyl mit 3 bis 8 Kohlenstoffatomen, oder für Phenyl, das einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen, oder
für Phenylalkyl mit 1 bis 4 Kohlenstoffatomen im Alkylteil, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen, oder
für Phenoxyalkyl mit 1 bis 4 Kohlenstoffatomen im Alkylteil, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 3 gleichen oder verschiedenen Halogenatomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen.
- R⁵: steht vorzugsweise für geradkettiges oder verzweigtes Alkyl mit 1 bis 6 Kohlenstoffatomen, Halogenalkyl mit 1 bis 6 Kohlenstoffatomen und 1 bis 7 gleichen oder verschiedenen Halogenatomen, Alkenyl mit 2 bis 6 Kohlenstoffatomen, Halogenalkenyl mit 2 bis 6 Kohlenstoffatomen und 1 bis 7 gleichen oder verschiedenen Halogenatomen, Alkoxyalkyl mit 1 bis 6 Kohlenstoffatomen im Alkylteil und 1 bis 6 Kohlenstoffatomen im Alkoxyteil, Alkoxyalkoxyalkyl mit 1 bis 6 Kohlenstoffatomen im Alkylteil und 1 bis 6 Kohlenstoffatomen in jedem Alkoxyteil, Alkylthioalkyl mit 1 bis 6 Kohlenstoffatomen im Alkylteil und 1 bis 6 Kohlenstoffatomen im Alkylthioteil, Alkylcarbonyloxyalkyl mit 1 bis 6 Kohlenstoffatomen im Alkylteil und 1 bis 6 Kohlenstoffatomen im Oxyalkylteil, Alkenylcarbonyloxyalkyl mit 2 bis 6 Kohlenstoffatomen im Alkenylteil und 1 bis 6 Kohlenstoffatomen im Oxyalkylteil oder für gegebenenfalls einfach bis dreifach, gleichartig oder verschieden durch Halogen und/oder Alkyl mit 1 bis 4 Kohlenstoffatomen substituiertes Cycloalkyl mit 3 bis 8 Kohlenstoffatomen, oder für Phenyl, das einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen, oder
für Phenylalkyl mit 1 bis 4 Kohlenstoffatomen im Alkylteil, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen, oder
für Phenoxyalkyl mit 1 bis 4 Kohlenstoffatomen im Alkylteil, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 3 gleichen oder verschiedenen Halogenatomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen.
- R: steht besonders bevorzugt für Fluor, Chlor, Brom, Iod, Trifluormethyl, Trichlormethyl, Difluormethyl, Fluormethyl und Fluorchlormethyl.
- R¹: steht besonders bevorzugt für Fluor, Chlor, Brom, Methyl, Ethyl, n-Propyl, Isopropyl, n-Butyl, tert.-Butyl, Trifluormethyl, Trichlormethyl, Difluormethyl, Fluormethyl und Fluorchlormethyl.
- m: steht besonders bevorzugt für ganze Zahlen von 2 bis 6, insbesondere für 4 oder 5.
- n: steht auch besonders bevorzugt für die Zahlen 0, 1, 2 oder 3.
- X¹: steht besonders bevorzugt für Wasserstoff, Fluor, Chlor, Brom, Iod, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und Trifluormethylthio.
- X²: steht besonders bevorzugt für Wasserstoff, Fluor, Chlor, Brom, Iod, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und Trifluormethylthio.
- X³: steht besonders bevorzugt für Wasserstoff, Fluor, Chlor, Brom, Iod, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und Trifluormethylthio.
- X⁴: steht besonders bevorzugt für Wasserstoff, Fluor, Chlor, Brom, Iod, Methyl, Ethyl, n-Propyl, Isopropyl, n-Butyl, tert.-Butyl, Trifluormethyl, Trichlormethyl, Difluormethyl, Fluormethyl, Fiuorchlormethyl, Methoxy, Ethoxy, Trifluormethoxy, Methylthio, Ethylthio und Trifluormethylthio.
- Z: steht auch besonders bevorzugt für Wasserstoff oder die Reste der Formeln 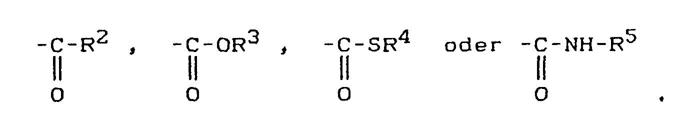
- R²: steht besonders bevorzugt für geradkettiges oder verzweigtes Alkyl mit 1 bis 4 Kohlenstoffatomen, Halogenalkyl mit 1 oder 2 Kohlenstoffatomen und 1 bis 3 Fluor-, Chlor- und/oder Bromatomen, Alkenyl mit 2 bis 4 Kohlenstoffatomen, Halogenalkenyl mit 2 bis 4 Kohlenstoffatomen und 1 bis 3 Fluor-, Chlor- und/oder Bromatomen, Alkoxyalkyl mit 1 bis 4 Kohlenstoffatomen im Alkylteil und 1 bis 4 Kohlenstoffatomen im Alkoxyteil, Alkoxyalkoxyalkyl mit 1 bis 4 Kohlenstoffatomen im Alkylteil und 1 bis 4 Kohlenstoffatomen in jedem Alkoxyteil, Alkylthioalkyl mit 1 bis 4 Kohlenstoffatomen im Alkylteil und 1 bis 4 Kohlenstoffatomen im Alkylthioteil, Alkylcarbonyloxyalkyl mit 1 bis 4 Kohlenstoffatomen im Alkylteil und 1 bis 4 Kohlenstoffatomen im Oxyalkylteil, Alkenylcarbonyloxyalkyl mit 2 bis 4 Kohlenstoffatomen im Alkenylteil und 1 bis 4 Kohlenstoffatomen im Oxyalkylteil, oder für gegebenenfalls einfach bis dreifach, gleichartig oder verschieden durch Fluor, Chlor, Brom, Methyl, Ethyl, n-Propyl, Isopropyl, n-Butyl und/oder tert. Butyl substituiertes Cycloalkyl mit 3 bis 7 Kohlenstoffatomen, oder
für Phenyl, das einfach bis dreifach, gleichartig oder verschieden substituiert sein kann durch Fluor, Chlor, Brom, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy Ethoxy, Trifluormethoxy, Methylthio, Ethylthio und/oder Trifluormethylthio, oder
Phenylalkyl mit 1 oder 2 Kohlenstoffatomen im Alkylteil, wobei der Phenylteil einfach bis dreifach, gleichartig oder verschieden substituiert sein kann durch Fluor, Chlor, Brom, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio, oder für Phenoxyalkyl mit 1 oder 2 Kohlenstoffatomen im Alkylteil, wobei der Phenylteil einfach bis dreifach, gleichartig oder verschieden substituiert sein kann durch Fluor, Chlor, Brom, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio.
- R³: steht besonders bevorzugt für geradkettiges oder verzweigtes Alkyl mit 1 bis 4 Kohlenstoffatomen, Halogenalkyl mit 1 oder 2 Kohlenstoffatomen und 1 bis 3 Fluor-, Chlor- und/oder Bromatomen, Alkenyl mit 2 bis 4 Kohlenstoffatomen, Halogenalkenyl mit 2 bis 4 Kohlenstoffatomen und 1 bis 3 Fluor-, Chlor- und/oder Bromatomen, Alkoxyalkyl mit 1 bis 4 Kohlenstoffatomen im Alkylteil und 1 bis 4 Kohlenstoffatomen im Alkoxyteil, Alkoxyalkoxyalkyl mit 1 bis 4 Kohlenstoffatomen im Alkylteil und 1 bis 4 Kohlenstoffatomen in jedem Alkoxyteil, Alkylthioalkyl mit 1 bis 4 Kohlenstoffatomen im Alkylteil und 1 bis 4 Kohlenstoffatomen im Alkylthioteil, Alkylcarbonyloxyalkyl mit 1 bis 4 Kohlenstoffatomen im Alkylteil und 1 bis 4 Kohlenstoffatomen im Oxyalkylteil, Alkenylcarbonyloxyalkyl mit 2 bis 4 Kohlenstoffatomen im Alkenylteil und 1 bis 4 Kohlenstoffatomen im Oxyalkylteil, oder für gegebenenfalls einfach bis dreifach, gleichartig oder verschieden durch Fluor, Chlor, Brom, Methyl, Ethyl, n-Propyl, Isopropyl, n-Butyl und/oder tert.-Butyl substituiertes Cycloalkyl mit 3 bis 7 Kohlenstoffatomen, oder
für Phenyl, das einfach bis dreifach, gleichartig oder verschieden substituiert sein kann durch Fluor, Chlor, Brom, Methyl, Ethyl, n-Propyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio, oder
für Phenylalkyl mit 1 oder 2 Kohlenstoffatomen im Alkylteil, wobei der Phenylteil einfach bis dreifach, gleichartig oder verschieden substituiert sein kann durch Fluor, Chlor, Brom, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio, oder für Phenoxyalkyl mit 1 oder 2 Kohlenstoffatomen im Alkylteil, wobei der Phenylteil einfach bis dreifach, gleichartig oder verschieden substituiert sein kann durch Fluor, Chlor, Brom, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio.
- R⁴: steht besonders bevorzugt für geradkettiges oder verzweigtes Alkyl mit 1 bis 4 Kohlenstoffatomen, Halogenalkyl mit 1 oder 2 Kohlenstoffatomen und 1 bis 3 Fluor-, Chlor- und/oder Bromatomen, Alkenyl mit 2 bis 4 Kohlenstoffatomen, Halogenalkenyl mit 2 bis 4 Kohlenstoffatomen und 1 bis 3 Fluor-, Chlor- und/oder Bromatomen, Alkoxyalkyl mit 1 bis 4 Kohlenstoffatomen im Alkylteil und 1 bis 4 Kohlenstoffatomen im Alkoxyteil, Alkoxyalkoxyalkyl mit 1 bis 4 Kohlenstoffatomen im Alkylteil und 1 bis 4 Kohlenstoffatomen in jedem Alkoxyteil, Alkylthioalkyl mit 1 bis 4 Kohlenstoffatomen im Alkylteil und 1 bis 4 Kohlenstoffatomen im Alkylthioteil, Alkylcarbonyloxyalkyl mit 1 bis 4 Kohlenstoffatomen im Alkylteil und 1 bis 4 Kohlenstoffatomen im Oxyalkylteil, Alkenylcarbonyloxyalkyl mit 2 bis 4 Kohlenstoffatomen im Alkenylteil und 1 bis 4 Kohlenstoffatomen im Oxyalkylteil, oder für gegebenenfalls einfach bis dreifach, gleichartig oder verschieden durch Fluor, Chlor, Brom, Methyl, Ethyl, n-Propyl, Isopropyl, n-Butyl und/oder tert.-Butyl substituiertes Cycloalkyl mit 3 bis 7 Kohlenstoffatomen, oder
für Phenyl, das einfach bis dreifach, gleichartig oder verschieden substituiert sein kann durch Fluor, Chlor, Brom, Methyl, Ethyl, n-Propyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio, oder
für Phenylalkyl mit 1 oder 2 Kohlenstoffatomen im Alkylteil, wobei der Phenylteil einfach bis dreifach, gleichartig oder verschieden substituiert sein kann durch Fluor, Chlor, Brom, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio, oder
für Phenoxyalkyl mit 1 oder 2 Kohlenstoffatomen im Alkylteil, wobei der Phenylteil einfach bis dreifach, gleichartig oder verschieden substituiert sein kann durch Fluor, Chlor, Brom, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio.
- R⁵: steht besonders bevorzugt für geradkettiges oder verzweigtes Alkyl mit 1 bis 4 Kohlenstoffatomen, Halogenalkyl mit 1 oder 2 Kohlenstoffatomen und 1 bis 3 Fluor-, Chlor- und/oder Bromatomen, Alkenyl mit 2 bis 4 Kohlenstoffatomen, Halogenalkenyl mit 2 bis 4 Kohlenstoffatomen und 1 bis 3 Fluor-, Chlor- und/oder Bromatomen, Alkoxyalkyl mit 1 bis 4 Kohlenstoffatomen im Alkylteil und 1 bis 4 Kohlenstoffatomen im Alkoxyteil, Alkoxyalkoxyalkyl mit 1 bis 4 Kohlenstoffatomen im Alkylteil und 1 bis 4 Kohlenstoffatomen in jedem Alkoxyteil, Alkylthioalkyl mit 1 bis 4 Kohlenstoffatomen im Alkylteil und 1 bis 4 Kohlenstoffatomen im Alkylthioteil, Alkylcarbonyloxyalkyl mit 1 bis 4 Kohlenstoffatomen im Alkylteil und 1 bis 4 Kohlenstoffatomen im Oxyalkylteil, Alkenylcarbonyloxyalkyl mit 2 bis 4 Kohlenstoffatomen im Alkenylteil und 1 bis 4 Kohlenstoffatomen im Oxyalkylteil, oder für gegebenenfalls einfach bis dreifach, gleichartig oder verschieden durch Fluor, Chlor, Brom, Methyl, Ethyl, n-Propyl, Isopropyl, n-Butyl und/oder tert.-Butyl substituiertes Cycloalkyl mit 3 bis 7 Kohlenstoffatomen, oder
für Phenyl, das einfach bis dreifach, gleichartig der verschieden substituiert sein kann durch
Fluor, Chlor, Brom, Methyl, Ethyl, n-Propyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio, oder
für Phenylalkyl mit 1 oder 2 Kohlenstoffatomen im Alkylteil, wobei der Phenylteil einfach bis dreifach, gleichartig oder verschieden substituiert sein kann durch Fluor, Chlor, Brom, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio, oder
für Phenoxyalkyl mit 1 oder 2 Kohlenstoffatomen im Alkylteil, wobei der Phenylteil einfach bis dreifach, gleichartig oder verschieden substituiert sein kann durch Fluor, Chlor, Brom, Methyl, Ethyl, n-Propyl, Isopropyl, n-Butyl, tert.-Butyl, Trifluormethyl, Trichlormethyl, Difluormethyl, Fluormethyl, Fluorchlormethyl, Methoxy, Ethoxy, Trifluormethoxy, Methylthio, Ethylthio und/oder Trifluormethylthio.

Von besonderem Interesse sind Verbindungen der Formel (I) in denen R¹, X³, X⁴ und Z die angegebenen Bedeutungen haben und in denen
- m: für die Zahlen 5 oder 6 steht,
- n: für die Zahl O steht,
- X¹: für Chlor steht,
- X²: für Chlor steht, und
- R: für Chlor, Brom oder Trifluormethyl steht.

Verwendet man 4-Amino-2,3-dichlorphenol und 1-Brom-cyclohexancarbonsäurechlorid als Ausgangsstoffe, so kann der Verlauf des erfindungsgemäßen Verfahrens (erste Stufe) durch das folgende Formelschema veranschaulicht werden:

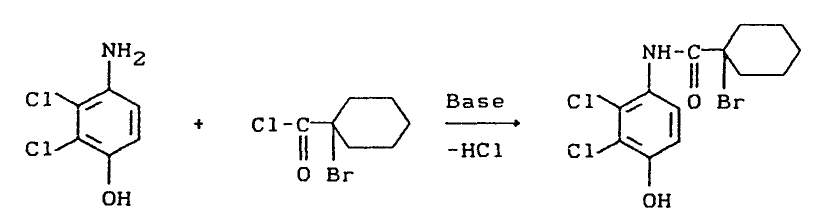

Verwendet man 4-(1-Brom-cyclohexanoyl)-amino-2,3-dichlor-phenol als Ausgangsstoff und Acetylchlorid als Reaktionskomponente, so kann der Verlauf des erfindungsgemäßen Verfahrens (zweite Stufe, Variante a) durch das folgende Formelschema veranschaulicht werden:

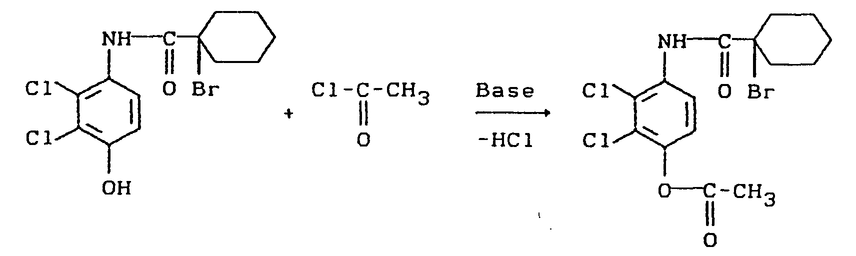

Verwendet man 4-(1-Brom-cyclohexanoyl)-amino-2,3-dichlor-phenol als Ausgangsstoff und Chlorameisensäuremethylester als Reaktionskomponente, so kann der Verlauf des erfindungsgemäßen Verfahrens (zweite Stufe, Variante b) durch das folgende Formelschema veranschaulicht werden:

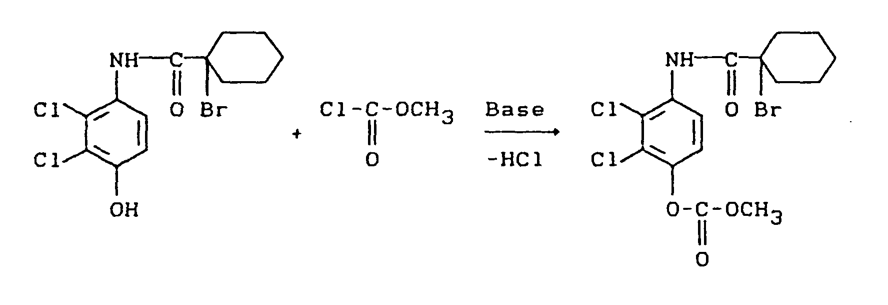

Verwendet man 4-(1-Brom-cyclohexanoyl)-amino-2,3-dichlor-phenol als Ausgangsstoff und Chlorameisensäurethioethylester als Reaktionskomponente, so kann der Verlauf des erfindungsgemäßen Verfahren (zweite Stufe, Variante c) durch das folgende Formelschema veranschaulicht werden:

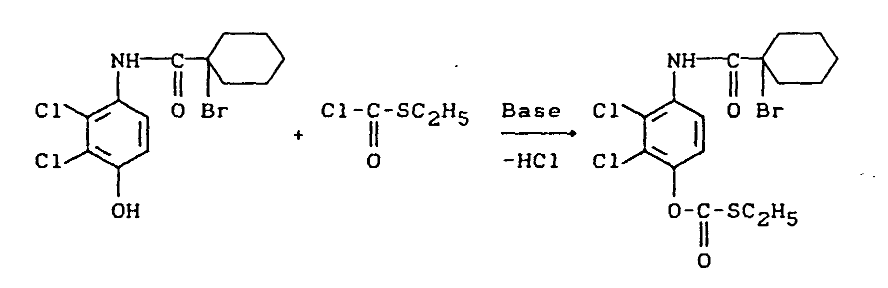

Verwendet man 4-(1-Brom-cyclohexanoyl)-amino-2,3-dichlor-phenol als Ausgangsstoff und 2-Methoxyethyl-isocyanat als Reaktionskomponente, so kann der Verlauf des erfindungsgemäßen Verfahrens (zweite Stufe, Variante d) durch das folgende Formelschema veranschaulicht werden:

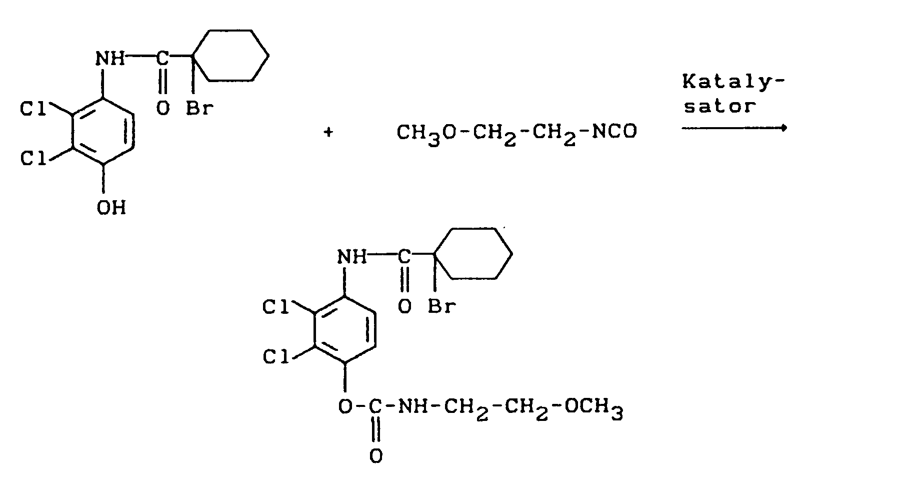

Die bei der Durchführung des erfindungsgemäßen Verfahrens (erste Stufe) als Ausgangsstoffe benötigten Aminophenole sind durch die Formel (II) allgemein definiert. In dieser Formel haben X¹, X², X³ und X⁴ vorzugsweise diejenigen Bedeutungen, die bereits im Zusammenhang mit der Beschreibung der erfindungsgemäßen Stoffe der Formel (I) für diese Reste als bevorzugt genannt wurden.

Die Aminophenole der Formel (II) sind bekannt oder lassen sich nach prinzipiell bekannten Methoden herstellen (vgl. EP-OS 0 416 365 und EP-OS 0 416 359).

Die bei der Durchführung des erfindungsgemäßen Verfahrens (erste Stufe) weiterhin als Ausgangsstoffe benötigten Cycloalkylcarbonsäure-Derivate sind durch die Formel (III) allgemein definiert. In dieser Formel haben R, R¹, m und n vorzugsweise diejenigen Bedeutungen, die bereits im Zusammenhang mit der Beschreibung der erfindungsgemäßen Stoffe der Formel (I) für diese Reste und Indices als bevorzugt genannt wurden. Y steht vorzugsweise für Chlor, Brom oder für einen Rest der Formel 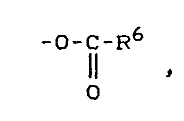 worin
R⁶ für Methyl, Ethyl oder 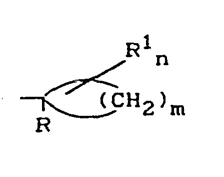 steht, wobei R, R¹, m und n die oben genannten Bedeutungen haben, oder
Y steht für die Reste der Formeln

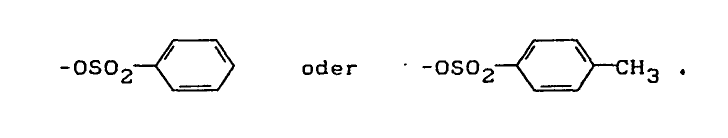

Die Cycloalkylcarbonsäure-Derivate der Formel (III) sind bekannt oder lassen sich nach prinzipiell bekannten Methoden herstellen (vgl. J. Amer. Chem. Soc. 81, 2126 (1959)).

Als Säurebindemittel kommen bei der Durchführung des erfindungsgemäßen Verfahrens (erste Stufe) alle üblichen anorganischen und organischen Säureakzeptoren in Frage. Vorzugsweise verwendbar sind Alkalimetallcarbonate und -alkoholate, wie Natrium- und Kaliumcarbonat, Natrium- und Kaliummethylat bzw. -ethylat, ferne aliphatische, aromatische und heterocyclische Amine, beispielsweise Triethylamin, Trimethylamin, Dimethylanilin, 1,8-Diazabicyclo-(4,5,0)-undec-7-en, Dimethylbenzylamin und Pyridin.

Als Verdünnungsmittel kommen bei der Durchführung des erfindungsgemäßen Verfahrens (erste Stufe) alle üblichen inerten organischen Solventien in Betracht. Vorzugsweise verwendbar sind aliphatische und aromatische, gegebenenfalls halogenierte Kohlenwasserstoffe wie Pentan, Hexan, Heptan, Cyclohexan, Petrolether, Benzin, Ligroin, Toluol, Xylol, Methylenchlorid, Ethylenchlroid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methylisopropyl- und Methylisobutylketon, Ester wie Essigsäuremethylester und -ethylester, Nitrile wie z.B. Acetonitril und Propionitril, Amide wie z.B. Dimethylformamid Dimethylacetamid und N-Methylpyrrolidon sowie Dimethylsulfoxid, Tetramethylensulfon und Hexamethylphosphorsäuretriamid.

Die Reaktionstemperaturen können bei der Durchführung des erfindungsgemäßen Verfahrens (erste Stufe) innerhalb eines größeren Bereiches variiert werden. Im allgemeinen arbeitet man bei Temperaturen zwischen -20°C und +120°C, vorzugsweise zwischen 0°C und +110°C.

Bei der Durchführung des erfindungsgemäßen Verfahrens arbeitet man sowohl in der ersten als auch in der zweiten Stufe (Varianten (a) bis (d)) im allgemeinen unter Normaldruck. Es ist jedoch auch möglich, jeweils unter erhöhtem oder vermindertem Druck zu arbeiten.

Bei der Durchführung der ersten Stufe des erfindungsgemäßen Verfahrens setzt man auf 1 Mol an Aminophenol der Formel (II) im allgemeinen 1 bis 2 Mol, vorzugsweise 1 bis 1,4 Mol an Cycloalkancarbonsäure-Derivat der Formel (III) ein. Die Aufarbeitung erfolgt nach üblichen Methoden.

Die bei der Durchführung der zweiten Stufe des erfindungsgemäßen Verfahrens als Ausgangsstoffe benötigten Cycloalkancarbonsäureanilide der Formel (Ia) sind erfindungsgemäße Stoffe.

Die bei der Durchführung der zweiten Stufe des erfindungsgemäßen Verfahrens nach Variante (a) als Reaktionskomponenten benötigten Carbonsäurehalogenide sind durch die Formel (IV) allgemein definiert. In dieser Formel hat R² vorzugsweise diejenigen Bedeutungen die bereits im Zusammenhang mit der Beschreibung der erfindungsgemäßen Stoffe der Formel (I) für diesen Rest als bevorzugt genannt wurden. Hal¹ steht vorzugsweise für Chlor oder Brom.

Die Carbonsäurehalogenide der Formel (IV) sind bekannt oder lassen sich nach prinzipiell bekannten Methoden herstellen.

Als Säurebindemittel kommen bei der Durchführung der zweiten Stufe des erfindungsgemäßen Verfahrens nach Variante (a) wiederum alle üblichen anorganischen und organischen Säureakzeptoren in Betracht. Vorzugsweise verwendbar sind diejenigen Basen, die schon im Zusammenhang mit der Durchführung der ersten Stufe des erfindungsgemäßen Verfahrens als bevorzugt genannt wurden.

Als Verdünnungsmittel kommen bei der Durchführung der zweiten Stufe des erfindungsgemäßen Verfahrens nach Variante (a) wiederum alle üblichen inerten organischen Solventien in Frage. Vorzugsweise verwendbar sind diejenigen Lösungsmittel, die schon im Zusammenhang mit der Durchführung der ersten Stufe des erfindungsgemäßen Verfahrens als bevorzugt genannt wurden.

Die Reaktionstemperaturen können auch bei der Durchführung der zweiten Stufe des erfindungsgemäßen Verfahrens nach Variante (a) innerhalb eines größeren Bereiches variiert werden. Im allgemeinen arbeitet man bei Temperaturen zwischen -20°C und +120°C, vorzugsweise zwischen 0°C und +110°C.

Bei der Durchführung der zweiten Stufe des erfindungsgemäßen Verfahrens nach Variante (a) setzt man auf 1 Mol an Cycloalkancarbonsäure-anilid der Formel (Ia) im allgemeinen 1 bis 2 Mol, vorzugsweise 1 bis 1,4 Mol an Carbonsäurehalogenid der Formel (IV) ein. Die Aufarbeitung erfolgt nach üblichen Methoden.

Die bei der Durchführung der zweiten Stufe des erfindungsgemäßen Verfahrens nach Variante (b) als Reaktionskomponenten benötigten Halogenameisensäureester sind durch die Formel (V) allgemein definiert. In dieser Formel hat R³ vorzugsweise diejenigen Bedeutungen die bereits im Zusammenhang mit der Beschreibung der erfindungsgemäßen Stoffe der Formel (I) für diesen Rest als bevorzugt genannt wurden. Hal² steht vorzugsweise für Chlor oder Brom.

Die Halogenameisensäureester der Formel (V) sind bekannt oder lassen sich nach prinzipiell bekannten Methoden herstellen.

Als Säurebindemittel kommen bei der Durchführung der zweiten Stufe des erfindungsgemäßen Verfahrens nach Variante (b) wiederum alle üblichen anorganischen und organischen Säureakzeptoren in Betracht. Vorzugsweise verwendbar sind diejenigen Basen, die schon im Zusammenhang mit der Durchführung der ersten Stufe des erfindungsgemäßen Verfahrens als bevorzugt genannt wurden.

Als Verdünnungsmittel kommen bei der Durchführung der zweiten Stufe des erfindungsgemäßen Verfahrens nach Variante (b) wiederum alle üblichen inerten organischen Solventien in Frage. Vorzugsweise verwendbar sind diejenigen Lösungsmittel, die schon im Zusammenhang mit der Durchführung der ersten Stufe des erfindungsgemäßen Verfahrens als bevorzugt genannt wurden.

Die Reaktionstemperaturen können auch bei der Durchführung der zweiten Stufe des erfindungsgemäßen Verfahrens nach Variante (b) innerhalb eines größeren Bereiches variiert werden. Im allgemeinen arbeitet man bei Temperaturen zwischen -20°C und +120°C, vorzugsweise zwischen 0°C und +110°C.

Bei der Durchführung der zweiten Stufe des erfindungsgemäßen Verfahrens nach Variante (b) setzt man auf 1 Mol an Cycloalkancarbonsäure-anilid der Formel (Ia) im allgemeinen 1 bis 2 Mol, vorzugsweise 1 bis 1,4 Mol an Halogenameisensäureester der Formel (V) ein. Die Aufarbeitung erfolgt nach üblichen Methoden.

Die bei der Durchführung der zweiten Stufe des erfindungsgemäßen Verfahrens nach Variante (c) als Reaktionskomponenten benötigten Halogenameisensäureester sind durch die Formel (VI) allgemein definiert. In dieser Formel hat R⁴ vorzugsweise diejenigen Bedeutungen die bereits im Zusammenhang mit der Beschreibung der erfindungsgemäßen Stoffe der Formel (I) für diesen Rest als bevorzugt genannt wurden. Hal³ steht vorzugsweise für Chlor oder Brom.

Die Halogenameisensäure-thioester der Formel (VI) sind bekannt oder lassen sich nach prinzipiell bekannten Methoden herstellen.

Als Säurebindemittel kommen bei der Durchführung der zweiten Stufe des erfindungsgemäßen Verfahrens nach Variante (c) wiederum alle üblichen anorganischen und organischen Säureakzeptoren in Betracht. Vorzugsweise verwendbar sind diejenigen Basen, die schon im Zusammenhang mit der Durchführung der ersten Stufe des erfindungsgemäßen Verfahrens als bevorzugt genannt wurden.

Als Verdünnungsmittel kommen bei der Durchführung der zweiten Stufe des erfindungsgemäßen Verfahrens nach Variante (c) wiederum alle üblichen inerten organischen Solventien in Frage. Vorzugsweise verwendbar sind diejenigen Lösungsmittel, die schon im Zusammenhang mit der Durchführung der ersten Stufe des erfindungsgemäßen Verfahrens als bevorzugt genannt wurden.

Die Reaktionstemperaturen können auch bei der Durchführung der zweiten Stufe des erfindungsgemäßen Verfahrens nach Variante (c) innerhalb eines größeren Bereiches variiert werden. Im allgemeinen arbeitet man bei Temperaturen zwischen -20°C und +120°C, vorzugsweise zwischen 0°C und +110°C.

Bei der Durchführung der zweiten Stufe des erfindungsgemäßen Verfahrens nach Variante (c) setzt man auf 1 Mol an Cycloalkancarbonsäure-anilid der Formel (Ia) im allgemeinen 1 bis 2 Mol, vorzugsweise 1 bis 1,4 Mol an Halogenameisensäure-thioester der Formel (VI) ein. Die Aufarbeitung erfolgt nach üblichen Methoden.

Die bei der Durchführung der zweiten Stufe des erfindungsgemäßen Verfahrens nach Variante (d) als Reaktionskomponenten benötigten Isocyanate sind durch die Formel (VII) allgemein definiert. In dieser Formel hat R⁵ vorzugsweise diejenigen Bedeutungen die bereits im Zusammenhang mit der Beschreibung der erfindungsgemäßen Stoffe der Formel (I) für diesen Rest als bevorzugt genannt wurden.

Die Isocyanate der Formel (VII) sind bekannt oder lassen sich nach prinzipiell bekannten Methoden herstellen.

Als Katalysatoren kommen bei der Durchführung der zweiten Stufe des erfindungsgemäßen Verfahrens nach Variante (d) alle für derartige Umsetzungen üblichen Reaktionsbeschleuniger in Betracht. Vorzugsweise verwendbar sind cyclische Amine, wie z.B. 1,8-Diazabicyclo[5,4,0]-undec-7-en.

Als Verdünnungsmittel kommen bei der Durchführung der zweiten Stufe des erfindungsgemäßen Verfahrens nach Variante (d) alle für derartige Umsetzungen üblichen, inerten organischen Solventien in Frage. Vorzugsweise verwendbar sind aliphatische und aromatische Kohlenwasserstoffe, wie Pentan, Hexan, Heptan, Cyclohexan, Petrolether, Benzin, Ligroin, Toluol, Xylol, oder Ether, wie Diethylether, Dibutylether, Glykoldimethylether, Diglykoldimethylether, Tetrahydrofuran und Dioxan.

Die Reaktionstemperaturen können auch bei der Durchführung der zweiten Stufe des erfindungsgemäßen Verfahrens nach Variante (d) innerhalb eines größeren Bereiches variiert werden. Im allgemeinen arbeitet man bei Temperaturen zwischen -20°C und +120°C, vorzugsweise zwischen 0°C und +110°C.

Bei der Durchführung der zweiten Stufe des erfindungsgemäßen Verfahrens nach Variante (d) setzt man auf 1 Mol an Cycloalkancarbonsäure-anilid der Formel (Ia) im allgemeinen 1 bis 2 Mol, vorzugsweise 1 bis 1,4 Mol an Isocyanat der Formel (VII) sowie eine geringe Menge an Katalysator ein. Die Aufarbeitung erfolgt nach üblichen Methoden.

Die erfindungsgemäßen Stoffe eignen sich als Schädlingsbekämpfungsmittel, insbesondere als Fungizide.

Fungizide werden im Pflanzenschutz eingesetzt zur Bekämpfung von Plasmadiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Botrytis. Sie zeigen außerdem auch eine gute in-vitro-Wirksamkeit.

Die erfindungsgemäßen Stoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nicht-ionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je kg Saatgut, vorzugsweise 0,01 bis 10 g benötigt. Bei der Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Stoffe geht aus den folgenden Beispielen hervor.

### Herstellungsbeispiele

### Beispiel 1

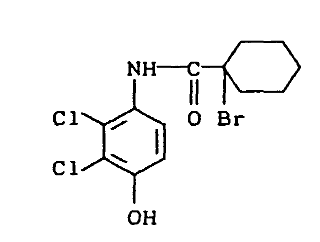

In ein Gemisch aus 10 g (0,056 Mol) 4-Amino-2,3-dichlorphenol und 100 ml Tetrahydrofuran werden bei 0°C unter Rühren 12,6 g (0,056 Mol) 1-Brom-cyclohexancarbonsäurechlorid eingetropft. Nach Zugabe der halben Menge (5 g) an Säurechlorid werden gleichzeitig 7,7 ml (0,05 Mol) Triethylamin zugetropft. Nach beendeter Zugabe wird das Reaktionsgemisch 24 Stunden bei 20°C gerührt und dann auf 300 ml Eiswasser gegossen. Man extrahiert zweimal mit je 300 ml Dichlormethan, trocknet die organische Phase über Magnesiumsulfat und engt durch Abziehen des Lösungsmittels unter vermindertem Druck ein. Der verbleibende Rückstand wird aus Toluol umkristallisiert. Man erhält auf diese Weise 11,9 g (58 % der Theorie) an 4-(1-Brom-cyclohexanoyl)-amino-2,3-dichlor-phenol in Form einer Festsubstanz vom Schmelzpunkt 142°C.

### Beispiel 2

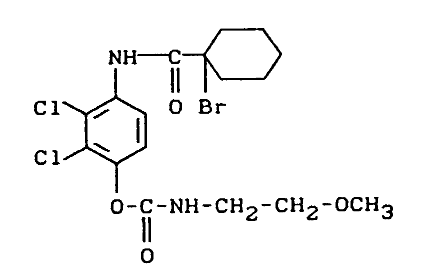

Eine Lösung von 2 g (5,5 mMol) 4-(1-Bromcyclohexanoyl)-amino-2,3-dichlor-phenol in 10 ml Toluol wird bei Raumtemperatur unter Rühren mit 0,6 g (6 mMol) 2-Methoxy-ethylisocyanat versetzt. Danach werden bei Raumtemperatur unter Rühren 30 mg (0,2 mMol) 1,8-Diaza-bicyclo[5,4,0]undec-7-en (DBU) hinzugefügt. Man rührt 24 Stunden bei 50°C nach und destilliert dann das Lösungsmittel unter vermindertem Druck ab. Der verbleibende Rückstand wird mit Toluol:Essigsäureethylester = 7:3 an Kieselgel chromatographiert. Man erhält auf diese Weise 1,6 g (64 % der Theorie) an 4-(2-Methoxy-ethyl-amino-carbonyloxy)-2,3-dichlor-(1-brom-cyclohexansäure)-anilid in Form einer Festsubstanz vom Schmelzpunkt 75°C.

### Beispiel 3

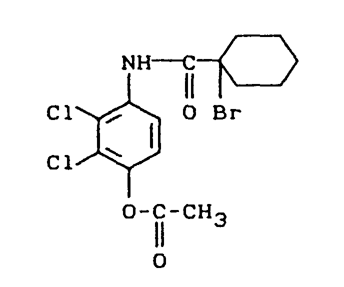

In ein Gemisch aus 2 g (5,5 mMol) 4-(1-Brom-cyclohexanoyl)-amino-2,3-dichlor-phenol, 0,8 ml (6 mMol) Triethylamin und 10 ml Tetrahydrofuran werden bei 20°C unter Rühren 0,5 g (6 mMol) Acetylchlorid langsam eingetropft. Nach beendeter Zugabe rührt man das Reaktionsgemisch 24 Stunden bei 20°C und gießt dann auf 100 ml Eiswasser. Der anfallende Feststoff wird abfiltriert, getrocknet und aus Toluol/Hexan umkristallisiert. Man erhält auf diese Weise 2 g (90 % der Theorie) an 4-Acetyloxy-2,3-dichlor-(1-brom-cyclohexansäure)-anilid in Form einer Festsubstanz vom Schmelzpunkt 129°C.

Nach den zuvor angegebenen Methoden werden auch die in der folgenden Tabelle aufgeführten Stoffe hergestellt.

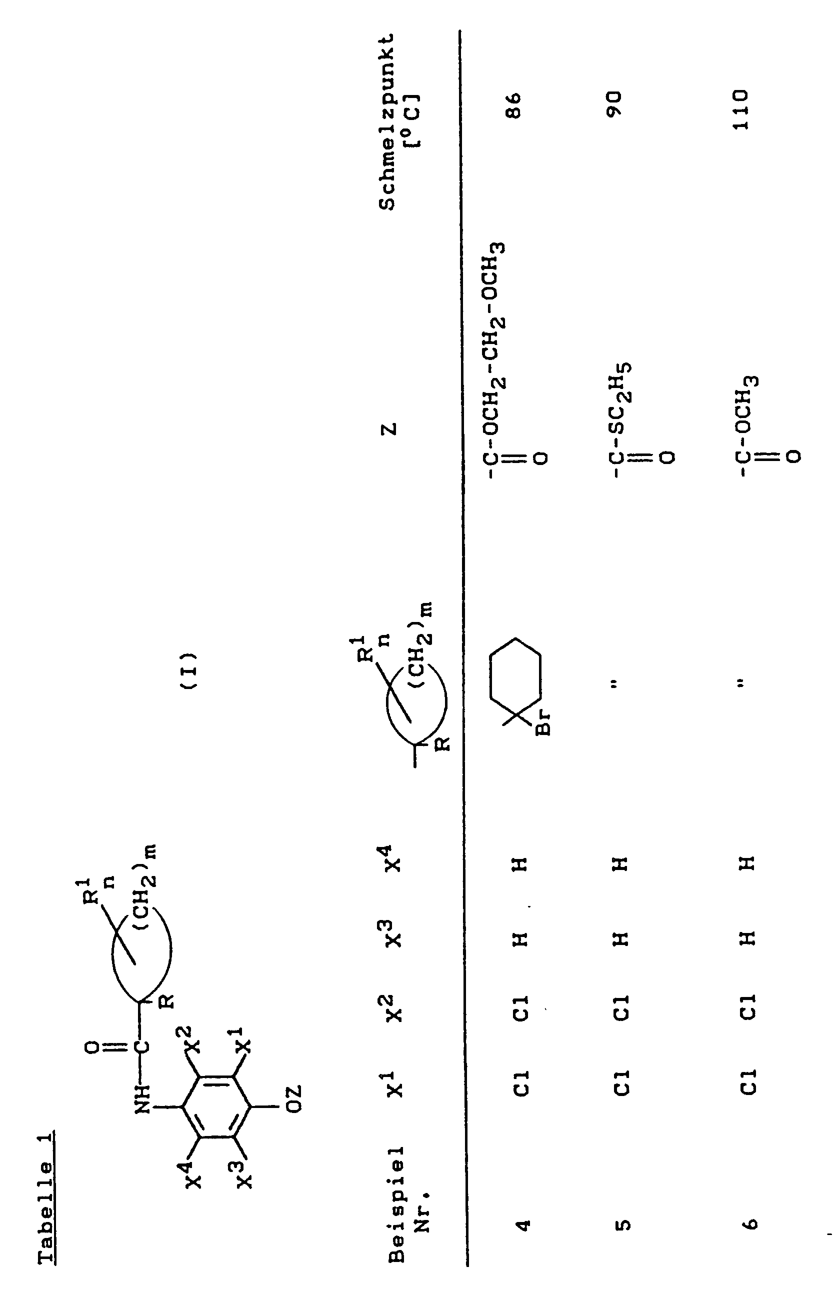

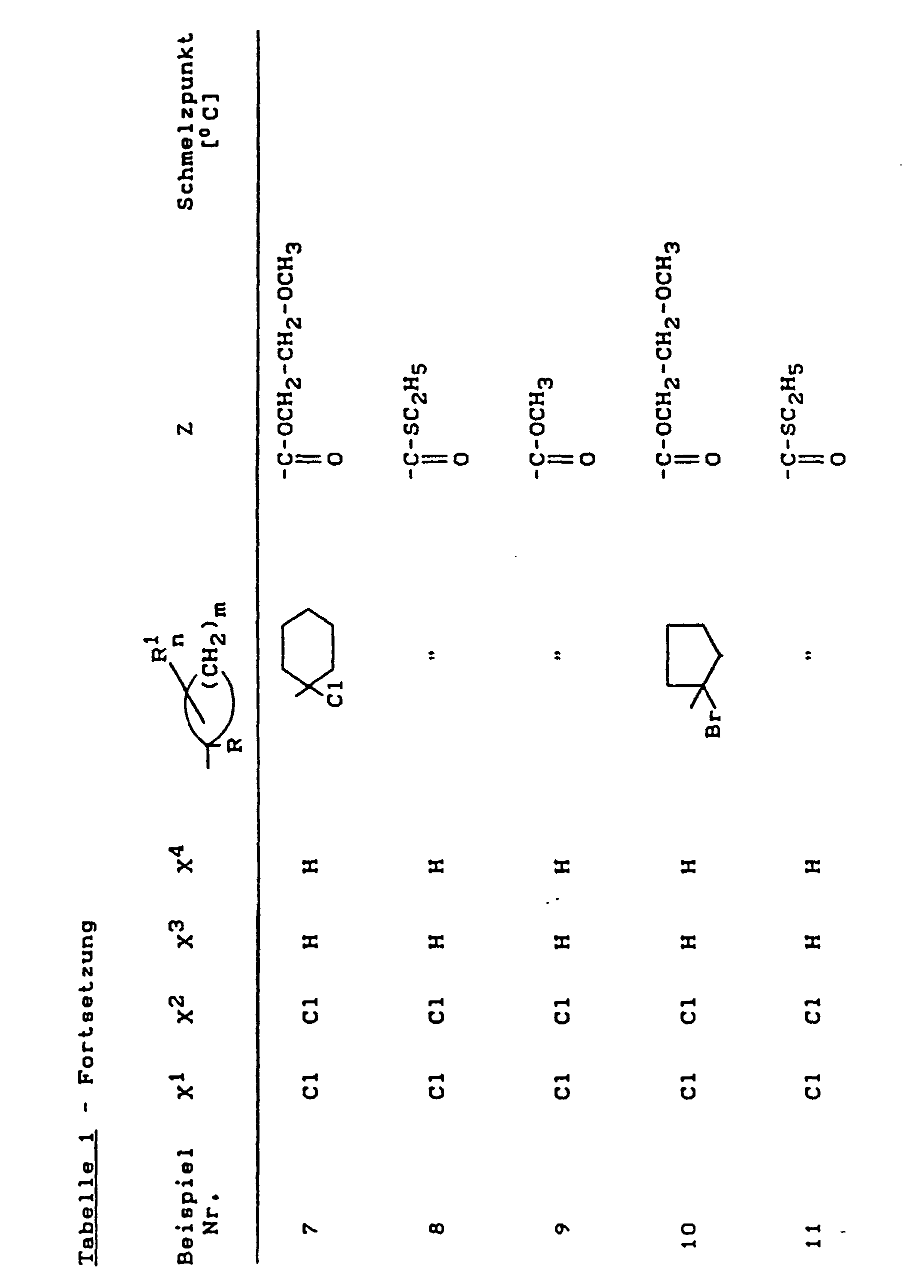

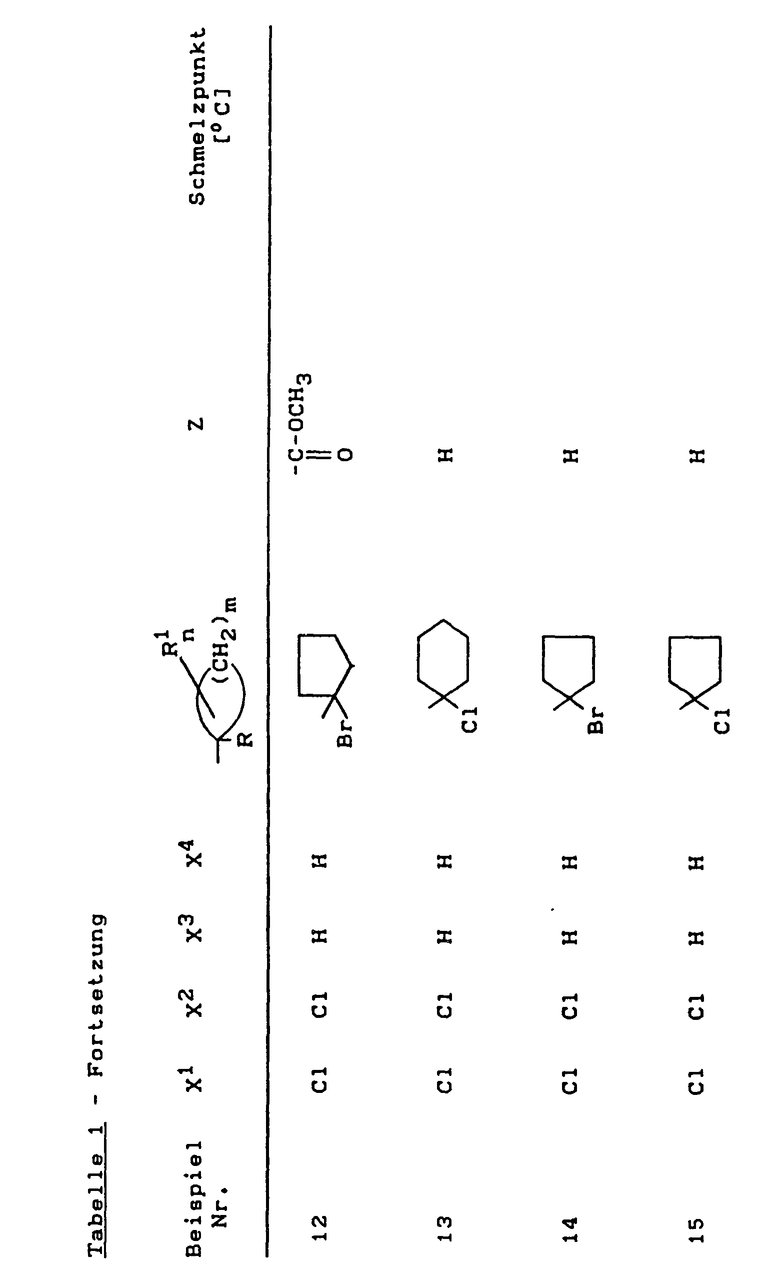

### Beispiel A

### Botrytis-Test (Buschbohne) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

In diesem Test zeigen die erfindungsgemäßen Stoffe (1) bis (6) bei einer Konzentration von 100 ppm in der Spritzflüssigkeit einen Wirkungsgrad von über 90 %.

## Patentansprüche

1. Cycloalkylcarbonsäure -anilide der Formel 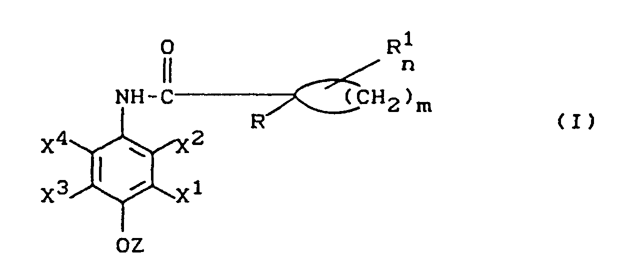 in welcher
R für Halogen oder Halogenalkyl steht,
R¹ für Halogen, Alkyl oder Halogenalkyl steht,
m für ganze Zahlen von 2 bis 7 steht,
n für die Zahlen 0, 1, 2 oder 3 steht,
X¹, X², X³ und X⁴ unabhängig voneinander für Wasserstoff, Halogen, Alkyl, Halogenalkyl, Alkoxy, Halogenalkoxy, Alkylthio oder Halogenalkylthio stehen und
Z für Wasserstoff oder die Reste der Formeln 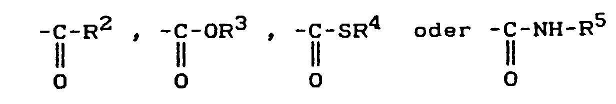 steht,
worin
R² für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht,
R³ für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht,
R⁴ für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht und
R⁵ für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht.

2. Cycloalkylcarbonsäure-anilide der Formel (I) gemäß Anspruch 1, in der
R für Fluor, Chlor, Brom, Iod oder Halogenalkyl mit 1 bis 3 Kohlenstoffatomen und 1 bis 5 gleichen oder verschiedenen Halogenatomen steht,
R¹ für Fluor, Chlor, Brom, geradkettiges oder verzweigtes Alkyl mit 1 bis 4 Kohlenstoffatomen oder für Halogenalkyl mit 1 bis 3 Kohlenstoffatomen und 1 bis 5 gleichen oder verschiedenen Halogenatomen steht,
m für ganze Zahlen von 2 bis 6 steht,
n für die Zahlen 0, 1, 2 und 3 steht,
X¹ für Wasserstoff, Fluor, Chlor, Brom, Iod, geradkettiges oder verzweigtes Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen oder für Halogenalkylthio mit 1 bis 3 Kohlenstoffatomen und 1 bis 5 gleichen oder verschiedenen Halogenatomen steht,
X² für Wasserstoff, Fluor, Chlor, Brom, Iod, geradkettiges oder verzweigtes Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen oder für Halogenalkylthio mit 1 bis 3 Kohlenstoffatomen und 1 bis 5 gleichen oder verschiedenen Halogenatomen steht,
X³ für Wasserstoff, Fluor, Chlor, Brom, Iod, geradkettiges oder verzweigtes Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen oder für Halogenalkylthio mit 1 bis 3 Kohlenstoffatomen und 1 bis 5 gleichen oder verschiedenen Halogenatomen steht,
X⁴ für Wasserstoff, geradkettiges oder verzweigtes Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen oder für Halogenalkylthio mit 1 bis 3 Kohlenstoffatomen und 1 bis 5 gleichen oder verschiedenen Halogenatomen steht,
und
Z für Wasserstoff oder die Reste der Formeln 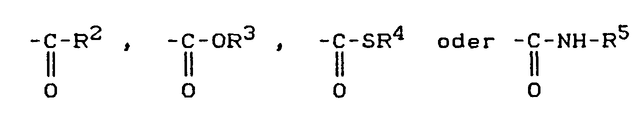 steht,
worin
R² für geradkettiges oder verzweigtes Alkyl mit 1 bis 6 Kohlenstoffatomen, Halogenalkyl mit 1 bis 6 Kohlenstoffatomen und 1 bis 7 gleichen oder verschiedenen Halogenatomen, Alkenyl mit 2 bis 6 Kohlenstoffatomen, Halogenalkenyl mit 2 bis 6 Kohlenstoffatomen und 1 bis 7 gleichen oder verschiedenen Halogenatomen, Alkoxyalkyl mit 1 bis 6 Kohlenstoffatomen im Alkylteil und 1 bis 6 Kohlenstoffatomen im Alkoxyteil, Alkoxyalkoxyalkyl mit 1 bis 6 Kohlenstoffatomen im Alkylteil und 1 bis 6 Kohlenstoffatomen in jedem Alkoxyteil, Alkylthioalkyl mit 1 bis 6 Kohlenstoffatomen im Alkylteil und 1 bis 6 Kohlenstoffatomen im Alkylthioteil, Alkylcarbonyloxyalkyl mit 1 bis 6 Kohlenstoffatomen im Alkylteil und 1 bis 6 Kohlenstoffatomen im Oxyalkylteil, Alkenylcarbonyloxyalkyl mit 2 bis 6 Kohlenstoffatomen im Alkenylteil und 1 bis 6 Kohlenstoffatomen im Oxyalkylteil oder für gegebenenfalls einfach bis dreifach, gleichartig oder verschieden durch Halogen und/oder Alkyl mit 1 bis 4 Kohlenstoffatomen substituiertes Cycloalkyl mit 3 bis 8 Kohlenstoffatomen steht, oder
für Phenyl steht, das einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen, oder
für Phenylalkyl mit 1 bis 4 Kohlenstoffatomen im Alkylteil steht, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen, oder
für Phenoxyalkyl mit 1 bis 4 Kohlenstoffatomen im Alkylteil steht, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 3 gleichen oder verschiedenen Halogen atomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen,
R³ für geradkettiges oder verzweigtes Alkyl mit 1 bis 6 Kohlenstoffatomen, Halogenalkyl mit 1 bis 6 Kohlenstoffatomen und 1 bis 7 gleichen oder verschiedenen Halogenatomen, Alkenyl mit 2 bis 6 Kohlenstoffatomen, Halogenalkenyl mit 2 bis 6 Kohlenstoffatomen und 1 bis 7 gleichen oder verschiedenen Halogenatomen, Alkoxyalkyl mit 1 bis 6 Kohlenstoffatomen im Alkylteil und 1 bis 6 Kohlenstoffatomen im Alkoxyteil, Alkoxyalkoxyalkyl mit 1 bis 6 Kohlenstoffatomen im Alkylteil und 1 bis 6 Kohlenstoffatomen in jedem Alkoxyteil, Alkylthioalkyl mit 1 bis 6 Kohlenstoffatomen im Alkylteil und 1 bis 6 Kohlenstoffatomen im Alkylthioteil, Alkylcarbonyloxyalkyl mit 1 bis 6 Kohlenstoffatomen im Alkylteil und 1 bis 6 Kohlenstoffatomen im Oxyalkylteil, Alkenylcarbonyloxyalkyl mit 2 bis 6 Kohlenstoffatomen im Alkenylteil und 1 bis 6 Kohlenstoffatomen im Oxyalkylteil oder für gegebenenfalls einfach bis dreifach, gleichartig oder verschieden durch Halogen und/oder Alkyl mit 1 bis 4 Kohlenstoffatomen substituiertes Cycloalkyl mit 3 bis 8 Kohlenstoffatomen steht, oder
für Phenyl steht, das einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/oder Halogenalkylthio mit 1 bis 3 Kohlenstoffatomen und 1 bis 5 gleichen oder verschiedenen Halogenatomen, oder
für Phenylalkyl mit 1 bis 4 Kohlenstoffatomen im Alkylteil steht, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen, oder
für Phenoxyalkyl mit 1 bis 4 Kohlenstoffatomen im Alkylteil steht, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 3 gleichen oder verschiedenen Halogen atomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen,
R⁴ für geradkettiges oder verzweigtes Alkyl mit 1 bis 6 Kohlenstoffatomen, Halogenalkyl mit 1 bis 6 Kohlenstoffatomen und 1 bis 7 gleichen oder verschiedenen Halogenatomen, Alkenyl mit 2 bis 6 Kohlenstoffatomen, Halogenalkenyl mit 2 bis 6 Kohlenstoffatomen und 1 bis 7 gleichen oder verschiedenen Halogenatomen, Alkoxyalkyl mit 1 bis 6 Kohlenstoffatomen im Alkylteil und 1 bis 6 Kohlenstoffatomen im Alkoxyteil, Alkoxyalkoxyalkyl mit 1 bis 6 Kohlenstoffatomen im Alkylteil und 1 bis 6 Kohlenstoffatomen in jedem Alkoxyteil, Alkylthioalkyl mit 1 bis 6 Kohlenstoffatomen im Alkylteil und 1 bis 6 Kohlenstoffatomen im Alkylthioteil, Alkylcarbonyloxyalkyl mit 1 bis 6 Kohlenstoffatomen im Alkylteil und 1 bis 6 Kohlenstoffatomen im Oxyalkylteil, Alkenylcarbonyloxyalkyl mit 2 bis 6 Kohlenstoffatomen im Alkenylteil und 1 bis 6 Kohlenstoffatomen im Oxyalkylteil oder für gegebenenfalls einfach bis dreifach, gleichartig oder verschieden durch Halogen und/oder Alkyl mit 1 bis 4 Kohlenstoffatomen substituiertes Cycloalkyl mit 3 bis 8 Kohlenstoffatomen steht, oder für Phenyl steht, das einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen, oder
für Phenylalkyl mit 1 bis 4 Kohlenstoffatomen im Alkylteil steht, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen, oder
für Phenoxyalkyl mit 1 bis 4 Kohlenstoffatomen im Alkylteil steht, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 3 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen und
R⁵ für geradkettiges oder verzweigtes Alkyl mit 1 bis 6 Kohlenstoffatomen, Halogenalkyl mit 1 bis 6 Kohlenstoffatomen und 1 bis 7 gleichen oder verschiedenen Halogenatomen, Alkenyl mit 2 bis 6 Kohlenstoffatomen, Halogenalkenyl mit 2 bis 6 Kohlenstoffatomen und 1 bis 7 gleichen oder verschiedenen Halogenatomen, Alkoxyalkyl mit 1 bis 6 Kohlenstoffatomen im Alkylteil und 1 bis 6 Kohlenstoffatomen im Alkoxyteil, Alkoxyalkoxyalkyl mit 1 bis 6 Kohlenstoffatomen im Alkylteil und 1 bis 6 Kohlenstoffatomen in jedem Alkoxyteil, Alkylthioalkyl mit 1 bis 6 Kohlenstoffatomen im Alkylteil und 1 bis 6 Kohlenstoffatomen im Alkylthioteil, Alkylcarbonyloxyalkyl mit 1 bis 6 Kohlenstoffatomen im Alkylteil und 1 bis 6 Kohlenstoffatomen im Oxyalkylteil, Alkenylcarbonyloxyalkyl mit 2 bis 6 Kohlenstoffatomen im Alkenylteil und 1 bis 6 Kohlenstoffatomen im Oxyalkylteil oder für gegebenenfalls einfach bis dreifach, gleichartig oder verschieden durch Halogen und/oder Alkyl mit 1 bis 4 Kohlenstoffatomen substituiertes Cycloalkyl mit 3 bis 8 Kohlenstoffatomen steht, oder für Phenyl steht, das einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen, oder
für Phenylalkyl mit 1 bis 4 Kohlenstoffatomen im Alkylteil steht, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen, oder
für Phenoxyalkyl mit 1 bis 4 Kohlenstoffatomen im Alkylteil steht, wobei der Phenylteil einfach bis dreifach, gleichartig oder verschieden substituiert sein kann durch Halogen, Alkyl mit 1 bis 4 Kohlenstoffatomen, Halogenalkyl mit 1 bis 3 Kohlenstoffatomen und 1 bis 5 gleichen oder verschiedenen Halogenatomen, Alkoxy mit 1 bis 4 Kohlenstoffatomen, Halogenalkoxy mit 1 bis 3 Kohlenstoffatomen und 1 bis 3 gleichen oder verschiedenen Halogenatomen, Alkylthio mit 1 bis 4 Kohlenstoffatomen und/ oder Halogenalkylthio mit 1 bis 3 Kohlenstoffatomen und 1 bis 5 gleichen oder verschiedenen Halogenatomen.

3. Verfahren zur Herstellung von Cycloalkylcarbonsäure-aniliden der Formel 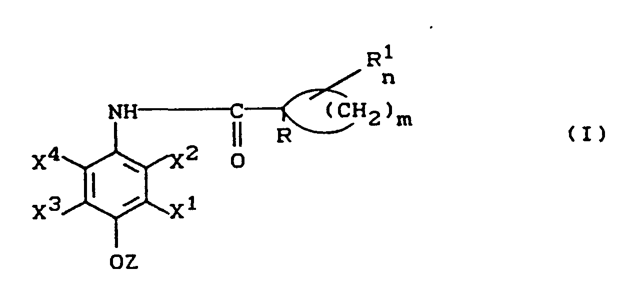 in welcher
R für Halogen oder Halogenalkyl steht,
R¹ für Halogen, Alkyl oder Halogenalkyl steht,
m für ganze Zahlen von 2 bis 7 steht,
n für die Zahlen 0, 1, 2 oder 3 steht,
X¹, X², X³ und X⁴ unabhängig voneinander für Wasserstoff, Halogen, Alkyl, Halogenalkyl, Alkoxy, Halogenalkoxy, Alkylthio oder Halogenalkylthio stehen und
Z für Wasserstoff oder die Reste der Formeln 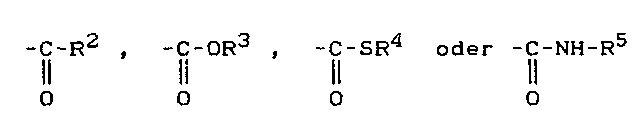 steht,
worin
R² für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht,
R³ für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht,
R⁴ für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht und
R⁵ für Alkyl, Halogenalkyl, Alkenyl, Halogenalkenyl, Alkoxyalkyl, Polyalkoxyalkyl, Alkylthioalkyl, Alkylcarbonyloxyalkyl, Alkenylcarbonyloxyalkyl, gegebenenfalls substituiertes Cycloalkyl, gegebenenfalls substituiertes Phenyl, für gegebenenfalls substituiertes Phenylalkyl oder für gegebenenfalls substituiertes Phenoxyalkyl steht,
dadurch gekennzeichnet, daß man Aminophenole der Formel 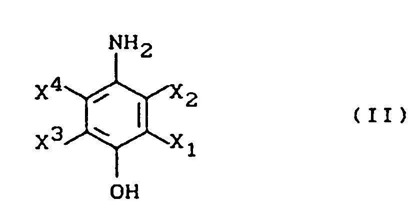 in welcher
X¹, X², X³ und X⁴ die oben angegebenen Bedeutungen haben,
mit Cycloalkylcarbonsäure-Derivaten der Formel 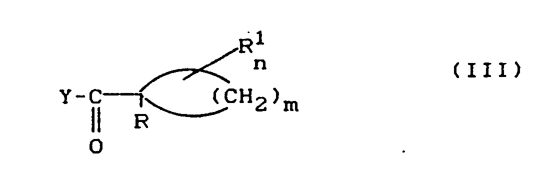 in welcher
R, R¹, m und n die oben angegebenen Bedeutungen haben und
Y für eine Leaving-Group steht,
gegebenenfalls in Gegenwart eines Säurebindemittels und gegebenenfalls in Gegenwart eines Verdünnungsmittels umsetzt und gegebenenfalls die dabei entstehenden Cycloalkylcarbonsäureanilide der Formel 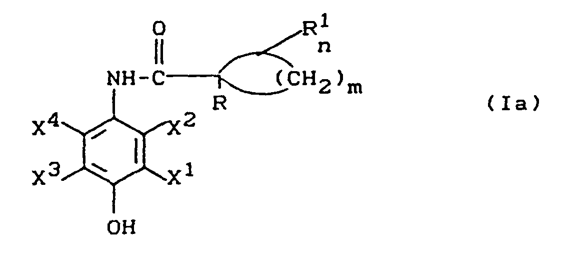 in welcher
R, R¹, m, n, X¹, X², X³ und X⁴ die oben angegebenen Bedeutungen haben,
entweder
a) mit Carbonsäurehalogeniden der Formel 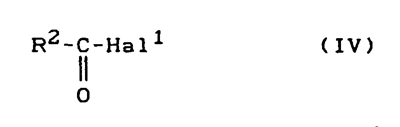 in welcher
R² die oben angegebene Bedeutung hat und
Hal¹ für Halogen steht,
gegebenenfalls in Gegenwart eines Säurebindemittels und gegebenenfalls in Gegenwart eines Verdünnungsmittels umsetzt,
oder
b) mit Halogenameisensäureestern der Formel 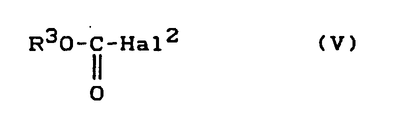 in welcher
R³ die oben angegebene Bedeutung hat und
Hal² für Halogen steht,
gegebenenfalls in Gegenwart eines Säurebindemittels und gegebenenfalls in Gegenwart eines Verdünnungsmittels umsetzt,
oder
c) mit Halogenameisensäure-thioestern der Formel 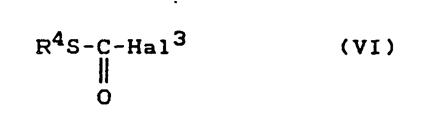 in welcher
R⁴ die oben angegebene Bedeutung hat und
Hal³ für Halogen steht,
gegebenenfalls in Gegenwart eines Säurebindemittels und gegebenenfalls in Gegenwart eines Verdünnungsmittels umsetzt,
oder
d) mit Isocyanaten der Formel
R⁵-NCO (VII)
in welcher
R⁵ die oben angegebene Bedeutung hat,
gegebenenfalls in Gegenwart eines Verdünnungsmittels und gegebenenfalls in Gegenwart eines Katalysators umsetzt.

4. Schädlingsbekämpfungsmittel, gekennzeichnet durch einen Gehalt an mindestens einem Cycloalkylcarbonsäureanilid der Formel (I) gemäß Anspruch 1.

5. Verwendung von Cycloalkylcarbonsäure-aniliden der Formel (I) gemäß Anspruch 1 zur Bekämpfung von Schädlingen.

6. Verfahren zur Bekämpfung von Schädlingen, dadurch gekennzeichnet, daß man Cycloalkylcarbonsäure-anilide der Formel (I) gemäß Anspruch 1 auf die Schädlinge und/oder deren Lebensraum ausbringt.

7. Verfahren zur Herstellung von Schädlingsbekämpfungsmitteln, dadurch gekennzeichnet, daß man Cycloalkylcarbonsäure-anilide der Formel (I) gemäß Anspruch 1 mit Streckmitteln und/oder oberflächenaktiven Stoffen vermischt.

## Claims

1. Cycloalkylcarboxanilides of the formula 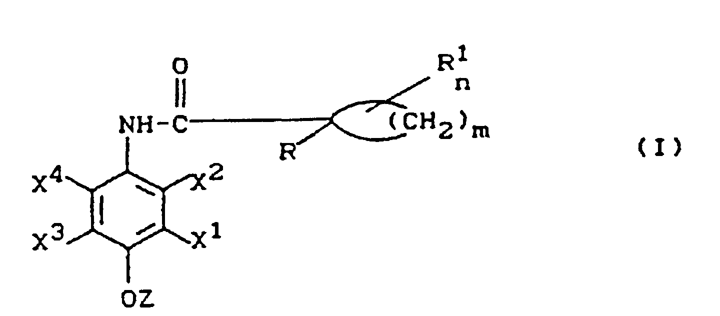 in which
R represents halogen or halogenoalkyl,
R¹ represents halogen, alkyl or halogenoalkyl,
m represents integers from 2 to 7,
n represents the numbers 0, 1, 2 or 3,
X¹, X², X³ and X⁴ independently of one another represent hydrogen, halogen, alkyl, halogenoalkyl, alkoxy, halogenoalkoxy, alkylthio or halogenoalkylthio, and
Z represents hydrogen or the radicals of the formulae 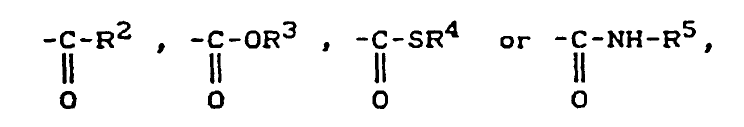 in which
R² represents alkyl, halogenoalkyl, alkenyl, halogenoalkenyl, alkoxyalkyl, polyalkoxyalkyl, alkylthioalkyl, alkylcarbonyloxyalkyl, alkenylcarbonyloxyalkyl, optionally substituted cycloalkyl, optionally substituted phenyl, optionally substituted phenylalkyl or optionally substituted phenoxyalkyl,
R³ represents alkyl, halogenoalkyl, alkenyl, halogenoalkenyl, alkoxyalkyl, polyalkoxyalkyl, alkylthioalkyl, alkylcarbonyloxyalkyl, alkenylcarbonyloxyalkyl, optionally substituted cycloalkyl, optionally substituted phenyl, optionally substituted phenylalkyl or optionally substituted phenoxyalkyl,
R⁴ represents alkyl, halogenoalkyl, alkenyl, halogenoalkenyl, alkoxyalkyl, polyalkoxyalkyl, alkylthioalkyl, alkylcarbonyloxyalkyl, alkenylcarbonyloxyalkyl, optionally substituted cycloalkyl, optionally substituted phenyl, optionally substituted phenylalkyl or optionally substituted phenoxyalkyl, and
R⁵ represents alkyl, halogenoalkyl, alkenyl, halogenoalkenyl, alkoxyalkyl, polyalkoxyalkyl, alkylthioalkyl, alkylcarbonyloxyalkyl, alkenylcarbonyloxyalkyl, optionally substituted cycloalkyl, optionally substituted phenyl, optionally substituted phenylalkyl or optionally substituted phenoxyalkyl,
have now been found.

2. Cycloalkylcarboxanilides of the formula (I) according to Claim 1, in which
R represents fluorine, chlorine, bromine, iodine or halogenoalkyl having 1 to 3 carbon atoms and 1 to 5 identical or different halogen atoms,
R¹ represents fluorine, chlorine, bromine, straight-chain or branched alkyl having 1 to 4 carbon atoms or halogenoalkyl having 1 to 3 carbon atoms and 1 to 5 identical or different halogen atoms,
m represents integers from 2 to 6,
n represents the numbers 0, 1, 2 and 3,
X¹ represents hydrogen, fluorine, chlorine, bromine, iodine, straight-chain or branched alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms or halogenoalkylthio having 1 to 3 carbon atoms and 1 to 5 identical or different halogen atoms,
X² represents hydrogen, fluorine, chlorine, bromine, iodine, straight-chain or branched alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms or halogenoalkylthio having 1 to 3 carbon atoms and 1 to 5 identical or different halogen atoms,
X³ represents hydrogen, fluorine, chlorine, bromine, iodine, straight-chain or branched alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms or halogenoalkylthio having 1 to 3 carbon atoms and 1 to 5 identical or different halogen atoms,
X⁴ represents hydrogen, straight-chain or branched alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms or halogenoalkylthio having 1 to 3 carbon atoms and 1 to 5 identical or different halogen atoms,
and
Z represents hydrogen or the radicals of the formulae 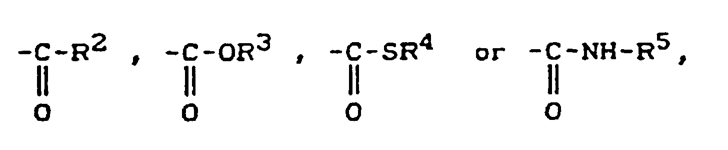 in which
R² represents straight-chain or branched alkyl having 1 to 6 carbon atoms, halogenoalkyl having 1 to 6 carbon atoms and 1 to 7 identical or different halogen atoms, alkenyl having 2 to 6 carbon atoms, halogenoalkenyl having 2 to 6 carbon atoms and 1 to 7 identical or different halogen atoms, alkoxyalkyl having 1 to 6 carbon atoms in the alkyl moiety and 1 to 6 carbon atoms in the alkoxy moiety, alkoxyalkoxyalkyl having 1 to 6 carbon atoms in the alkyl moiety and 1 to 6 carbon atoms in each alkoxy moiety, alkylthioalkyl having 1 to 6 carbon atoms in the alkyl moiety and 1 to 6 carbon atoms in the alkylthio moiety, alkylcarbonyloxyalkyl having 1 to 6 carbon atoms in the alkyl moiety and 1 to 6 carbon atoms in the oxyalkyl moiety, alkenylcarbonyloxyalkyl having 2 to 6 carbon atoms in the alkenyl moiety and 1 to 6 carbon atoms in the oxyalkyl moiety, or represents cycloalkyl having 3 to 8 carbon atoms which is optionally monosubstituted to trisubstituted by identical or different substituents from the series consisting of halogen and/or alkyl having 1 to 4 carbon atoms, or
represents phenyl which can be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms and/or halogenoalkylthio having 1 to 3 carbon atoms and 1 to 5 identical or different halogen atoms, or
represents phenylalkyl having 1 to 4 carbon atoms in the alkyl moiety, it being possible for the phenyl moiety to be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms and/or halogenoalkylthio having 1 to 3 carbon atoms and 1 to 5 identical or different halogen atoms, or
represents phenoxyalkyl having 1 to 4 carbon atoms in the alkyl moiety, it being possible for the phenyl moiety to be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 3 identical or different halogen atoms, alkylthio having 1 to 4 carbon atoms and/or halogenoalkylthio having 1 to 3 carbon atoms and 1 to 5 identical or different halogen atoms,
R³ represents straight-chain or branched alkyl having 1 to 6 carbon atoms, halogenoalkyl having 1 to 6 carbon atoms and 1 to 7 identical or different halogen atoms, alkenyl having 2 to 6 carbon atoms, halogenoalkenyl having 2 to 6 carbon atoms and 1 to 7 identical or different halogen atoms, alkoxyalkyl having 1 to 6 carbon atoms in the alkyl moiety and 1 to 6 carbon atoms in the alkoxy moiety, alkoxyalkoxyalkyl having 1 to 6 carbon atoms in the alkyl moiety and 1 to 6 carbon atoms in each alkoxy moiety, alkylthioalkyl having 1 to 6 carbon atoms in the alkyl moiety and 1 to 6 carbon atoms in the alkylthio moiety, alkylcarbonyloxyalkyl having 1 to 6 carbon atoms in the alkyl moiety and 1 to 6 carbon atoms in the oxyalkyl moiety, alkenylcarbonyloxyalkyl having 2 to 6 carbon atoms in the alkenyl moiety and 1 to 6 carbon atoms in the oxyalkyl moiety, or represents cycloalkyl having 3 to 8 carbon atoms which is optionally monosubstituted to trisubstituted by identical or different substituents from the series consisting of halogen and/or alkyl having 1 to 4 carbon atoms, or
represents phenyl which can be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms and/or halogenoalkylthio having 1 to 3 carbon atoms and 1 to 5 identical or different halogen atoms, or
represents phenylalkyl having 1 to 4 carbon atoms in the alkyl moiety, it being possible for the phenyl moiety to be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms and/or halogenoalkylthio having 1 to 3 carbon atoms and 1 to 5 identical or different halogen atoms, or
represents phenoxyalkyl having 1 to 4 carbon atoms in the alkyl moiety, it being possible for the phenyl moiety to be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 3 identical or different halogen atoms, alkylthio having 1 to 4 carbon atoms and/or halogenoalkylthio having 1 to 3 carbon atoms and 1 to 5 identical or different halogen atoms,
R⁴ represents straight-chain or branched alkyl having 1 to 6 carbon atoms, halogenoalkyl having 1 to 6 carbon atoms and 1 to 7 identical or different halogen atoms, alkenyl having 2 to 6 carbon atoms, halogenoalkenyl having 2 to 6 carbon atoms and 1 to 7 identical or different halogen atoms, alkoxyalkyl having 1 to 6 carbon atoms in the alkyl moiety and 1 to 6 carbon atoms in the alkoxy moiety, alkoxyalkoxyalkyl having 1 to 6 carbon atoms in the alkyl moiety and 1 to 6 carbon atoms in each alkoxy moiety, alkylthioalkyl having 1 to 6 carbon atoms in the alkyl moiety and 1 to 6 carbon atoms in the alkylthio moiety, alkylcarbonyloxyalkyl having 1 to 6 carbon atoms in the alkyl moiety and 1 to 6 carbon atoms in the oxyalkyl moiety, alkenylcarbonyloxyalkyl having 2 to 6 carbon atoms in the alkenyl moiety and 1 to 6 carbon atoms in the oxyalkyl moiety, or represents cycloalkyl having 3 to 8 carbon atoms which is optionally monosubstituted to trisubstituted by identical or different substituents from the series consisting of halogen and/or alkyl having 1 to 4 carbon atoms, or represents phenyl which can be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms and/or halogenoalkylthio having 1 to 3 carbon atoms and 1 to 5 identical or different halogen atoms, or
represents phenylalkyl having 1 to 4 carbon atoms in the alkyl moiety, it being possible for the phenyl moiety to be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms and/or halogenoalkylthio having 1 to 3 carbon atoms and 1 to 5 identical or different halogen atoms, or
represents phenoxyalkyl having 1 to 4 carbon atoms in the alkyl moiety, it being possible for the phenyl moiety to be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 3 identical or different halogen atoms, alkylthio having 1 to 4 carbon atoms and/or halogenoalkylthio having 1 to 3 carbon atoms and 1 to 5 identical or different halogen atoms, and
R⁵ represents straight-chain or branched alkyl having 1 to 6 carbon atoms, halogenoalkyl having 1 to 6 carbon atoms and 1 to 7 identical or different halogen atoms, alkenyl having 2 to 6 carbon atoms, halogenoalkenyl having 2 to 6 carbon atoms and 1 to 7 identical or different halogen atoms, alkoxyalkyl having 1 to 6 carbon atoms in the alkyl moiety and 1 to 6 carbon atoms in the alkoxy moiety, alkoxyalkoxyalkyl having 1 to 6 carbon atoms in the alkyl moiety and 1 to 6 carbon atoms in each alkoxy moiety, alkylthioalkyl having 1 to 6 carbon atoms in the alkyl moiety and 1 to 6 carbon atoms in the alkylthio moiety, alkylcarbonyloxyalkyl having 1 to 6 carbon atoms in the alkyl moiety and 1 to 6 carbon atoms in the oxyalkyl moiety, alkenylcarbonyloxyalkyl having 2 to 6 carbon atoms in the alkenyl moiety and 1 to 6 carbon atoms in the oxyalkyl moiety, or represents cycloalkyl having 3 to 8 carbon atoms which is optionally monosubstituted to trisubstituted by identical or different substituents from the series consisting of halogen and/or alkyl having 1 to 4 carbon atoms, or represents phenyl which can be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms and/or halogenoalkylthio having 1 to 3 carbon atoms and 1 to 5 identical or different halogen atoms, or
represents phenylalkyl having 1 to 4 carbon atoms in the alkyl moiety, it being possible for the phenyl moiety to be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 5 identical or different halogen atoms, alkylthio having 1 to 4 carbon atoms and/or halogenoalkylthio having 1 to 3 carbon atoms and 1 to 5 identical or different halogen atoms, or
represents phenoxyalkyl having 1 to 4 carbon atoms in the alkyl moiety, it being possible for the phenyl moiety to be monosubstituted to trisubstituted by identical or different substituents from the series consisting of halogen, alkyl having 1 to 4 carbon atoms, halogenoalkyl having 1 to 3 carbon atoms and 1 to 5 identical or different halogen atoms, alkoxy having 1 to 4 carbon atoms, halogenoalkoxy having 1 to 3 carbon atoms and 1 to 3 identical or different halogen atoms, alkylthio having 1 to 4 carbon atoms and/or halogenoalkylthio having 1 to 3 carbon atoms and 1 to 5 identical or different halogen atoms.

3. Process for the preparation of cycloalkylcarboxanilides of the formula 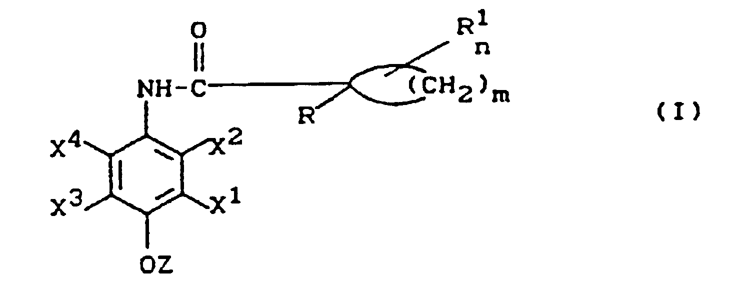 in which
R represents halogen or halogenoalkyl,
R¹ represents halogen, alkyl or halogenoalkyl,
m represents integers from 2 to 7,
n represents the numbers 0, 1, 2 or 3,
X¹, X², X³ and X⁴ independently of one another represent hydrogen, halogen, alkyl, halogenoalkyl, alkoxy, halogenoalkoxy, alkylthio or halogenoalkylthio, and
Z represents hydrogen or the radicals of the formulae 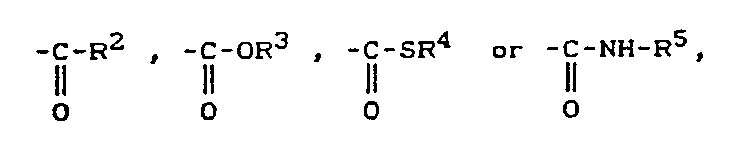 in which
R² represents alkyl, halogenoalkyl, alkenyl, halogenoalkenyl, alkoxyalkyl, polyalkoxyalkyl, alkylthioalkyl, alkylcarbonyloxyalkyl, alkenylcarbonyloxyalkyl, optionally substituted cycloalkyl, optionally substituted phenyl, optionally substituted phenylalkyl or optionally substituted phenoxyalkyl,
R³ represents alkyl, halogenoalkyl, alkenyl, halogenoalkenyl, alkoxyalkyl, polyalkoxyalkyl, alkylthioalkyl, alkylcarbonyloxyalkyl, alkenylcarbonyloxyalkyl, optionally substituted cycloalkyl, optionally substituted phenyl, optionally substituted phenylalkyl or optionally substituted phenoxyalkyl,
R⁴ represents alkyl, halogenoalkyl, alkenyl, halogenoalkenyl, alkoxyalkyl, polyalkoxyalkyl, alkylthioalkyl, alkylcarbonyloxyalkyl, alkenylcarbonyloxyalkyl, optionally substituted cycloalkyl, optionally substituted phenyl, optionally substituted phenylalkyl or optionally substituted phenoxyalkyl and,
R⁵ represents alkyl, halogenoalkyl, alkenyl, halogenoalkenyl, alkoxyalkyl, polyalkoxyalkyl, alkylthioalkyl, alkylcarbonyloxyalkyl, alkenylcarbonyloxyalkyl, optionally substituted cycloalkyl, optionally substituted phenyl, optionally substituted phenylalkyl or optionally substituted phenoxyalkyl,
characterized in that aminophenols of the formula 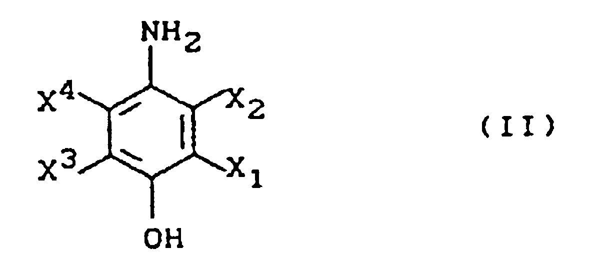 in which
X¹, X², X³ and X⁴ have the abovementioned meanings,
are reacted with cycloalkylcarboxylic acid derivatives of the formula 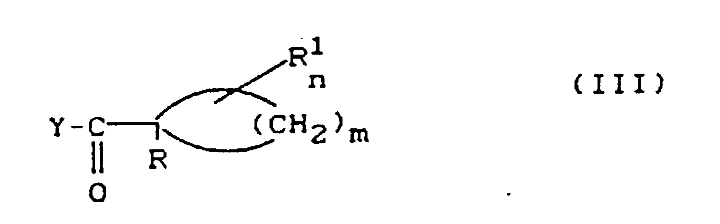 in which
R, R¹, m and n have the abovementioned meanings and
Y represents a leaving group,
if appropriate in the presence of an acid-binding agent and if appropriate in the presence of a diluent and, if appropriate, the resulting cycloalkylcarboxanilides of the formula 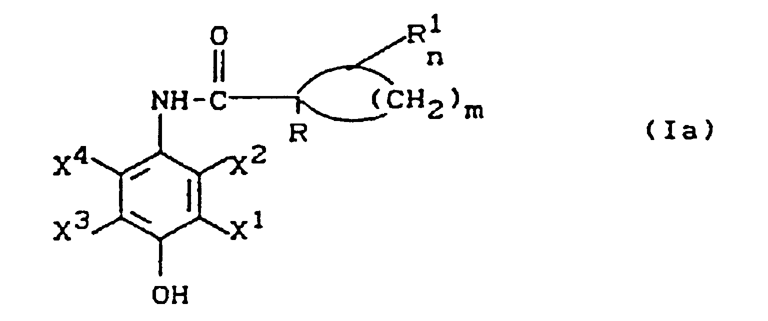 in which
R, R¹, m, n, X¹, X², X³ and X⁴ have the abovementioned meanings,
are reacted either
a) with carboxylic acid halides of the formula 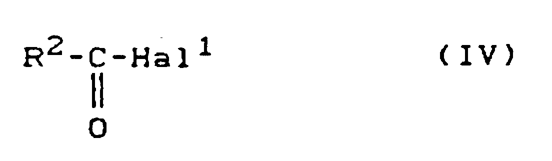 in which
R² has the abovementioned meaning and
Hal¹ represents halogen,
if appropriate in the presence of an acid-binding agent and if appropriate in the presence of a diluent,
or
b) with halogenoformic esters of the formula 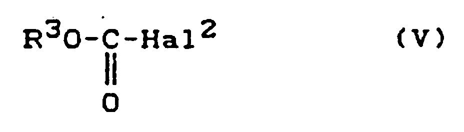 in which
R³ has the abovementioned meaning and
Hal² represents halogen,
if appropriate in the presence of an acid-binding agent and if appropriate in the presence of a diluent,
or
c) with halogenoformic thioesters of the formula 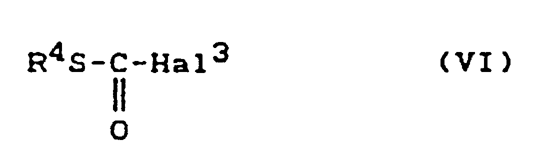 in which
R⁴ has the abovementioned meaning and
Hal³ represents halogen,
if appropriate in the presence of an acid-binding agent and if appropriate in the presence of a diluent,
or
d) with isocyanates of the formula
R⁵-NCO (VII)
in which
R⁵ has the abovementioned meaning,
if appropriate in the presence of a diluent and
if appropriate in the presence of a catalyst.

4. Pesticides, characterized in that they contain at least one cycloalkylcarboxanilide of the formula (I) according to Claim 1.

5. Use of cycloalkylcarboxanilides of the formula (I) according to Claim 1 for combating pests.

6. Method of combating pests, characterized in that cycloalkylcarboxanilides of the formula (I) according to Claim 1 are applied to the pests and/or their environment.

7. Process for the preparation of pesticides, characterized in that cycloalkylcarboxanilides of the formula (I) according to Claim 1 are mixed with extenders and/or surface-active substances.

## Revendications

1. Anilides d'acides cycloalkylcarboxyliques répondant à la formule 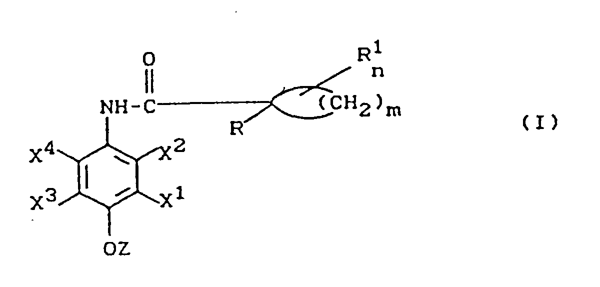 dans laquelle
R représente un atome d'halogène ou un groupe halogénalkyle,
R¹ représente un atome d'halogène, un groupe alkyle ou un groupe halogénalkyle,
m représente des nombres entiers de 2 à 7,
n représente les nombres 0, 1, 2 ou 3,
X¹, X², X³ et X⁴ représentent, indépendamment l'un de l'autre un atome d'hydrogène, un atome d'halogène, un groupe alkyle, un groupe halogénalkyle, un groupe alcoxy, un groupe halogénalcoxy, un groupe alkylthio ou un groupe halogénalkylthio, et
Z représente un atome d'hydrogène ou les radicaux répondant aux formules 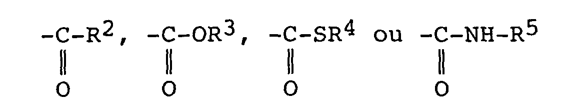 dans lesquelles
R² représente un groupe alkyle, un groupe halogénalkyle, un groupe alcényle, un groupe halogénalcényle, un groupe alcoxyalkyle, un groupe polyalcoxyalkyle, un groupe alkylthioalkyle, un groupe alkylcarbonyloxyalkyle, un groupe alcénylcarbonyloxyalkyle, un groupe cycloalkyle le cas échéant substitué, un groupe phényle le cas échéant substitué, représente un groupe phénylalkyle le cas échéant substitué ou représente un groupe phénoxyalkyle le cas échéant substitué,
R³ représente un groupe alkyle, un groupe halogénalkyle, un groupe alcényle, un groupe halogénalcényle, un groupe alcoxyalkyle, un groupe polyalcoxyalkyle, un groupe alkylthioalkyle, un groupe alkylcarbonyloxyalkyle, un groupe alcénylcarbonyloxyalkyle, un groupe cycloalkyle le cas échéant substitué, un groupe phényle le cas échéant substitué, représente un groupe phénylalkyle le cas échéant substitué ou représente un groupe phénoxyalkyle le cas échéant substitué,
R⁴ représente un groupe alkyle, un groupe halogénalkyle, un groupe alcényle, un groupe halogénalcényle, un groupe alcoxyalkyle, un groupe polyalcoxyalkyle, un groupe alkylthioalkyle, un groupe alkylcarbonyloxyalkyle, un groupe alcénylcarbonyloxyalkyle, un groupe cycloalkyle le cas échéant substitué, un groupe phényle le cas échéant substitué, représente un groupe phénylalkyle le cas échéant substitué ou représente un groupe phénoxyalkyle le cas échéant substitué, et
R⁵ représente un groupe alkyle, un groupe halogénalkyle, un groupe alcényle, un groupe halogénalcényle, un groupe alcoxyalkyle, un groupe polyalcoxyalkyle, un groupe alkylthioalkyle, un groupe alkylcarbonyloxyalkyle, un groupe alcénylcarbonyloxyalkyle, un groupe cycloalkyle le cas échéant substitué, un groupe phényle le cas échéant substitué, représente un groupe phénylalkyle le cas échéant substitué ou représente un groupe phénoxyalkyle le cas échéant substitué.

2. Anilides d'acides cycloalkylcarboxyliques répondant à la formule (I) selon la revendication 1, dans lesquels
R représente un atome de fluor, un atome de chlore, un atome de brome, un atome d'iode ou un groupe halogénalkyle contenant de 1 à 3 atomes de carbone et de 1 à 5 atomes d'halogène identiques ou différents,
R¹ représente un atome de fluor, un atome de chlore, un atome de brome, un groupe alkyle à chaîne droite ou ramifiée contenant de 1 à 4 atomes de carbone ou représente un groupe halogénalkyle contenant de 1 à 3 atomes de carbone et de 1 à 5 atomes d'halogène identiques ou différents,
m représente des nombres entiers de 2 à 6,
n représente les nombres 0, 1, 2 et 3,
X¹ représente un atome d'hydrogène, un atome de fluor, un atome de chlore, un atome de brome, un atome d'iode, un groupe alkyle à chaîne droite ou ramifiée contenant de 1 à 4 atomes de carbone, un groupe halogénalkyle contenant de 1 à 3 atomes de carbone et de 1 à 5 atomes d'halogène identiques ou différents, un groupe alcoxy contenant de 1 à 4 atomes de carbone, un groupe halogénalcoxy contenant de 1 à 3 atomes de carbone et de 1 à 5 atomes d'halogène identiques ou différents, un groupe alkylthio contenant de 1 à 4 atomes de carbone ou représente un groupe halogénalkylthio contenant de 1 à 3 atomes de carbone et de 1 à 5 atomes d'halogène identiques ou différents,
X² représente un atome d'hydrogène, un atome de fluor, un atome de chlore, un atome de brome, un atome d'iode, un groupe alkyle à chaîne droite ou ramifiée contenant de 1 à 4 atomes de carbone, un groupe halogénalkyle contenant de 1 à 3 atomes de carbone et de 1 à 5 atomes d'halogène identiques ou différents, un groupe alcoxy contenant de 1 à 4 atomes de carbone, un groupe halogénalcoxy contenant de 1 à 3 atomes de carbone et de 1 à 5 atomes d'halogène identiques ou différents, un groupe alkylthio contenant de 1 à 4 atomes de carbone ou représente un groupe halogénalkylthio contenant de 1 à 3 atomes de carbone et de 1 à 5 atomes d'halogène identiques ou différents,
X³ représente un atome d'hydrogène, un atome de fluor, un atome de chlore, un atome de brome, un atome d'iode, un groupe alkyle à chaîne droite ou ramifiée contenant de 1 à 4 atomes de carbone, un groupe halogénalkyle contenant de 1 à 3 atomes de carbone et de 1 à 5 atomes d'halogène identiques ou différents, un groupe alcoxy contenant de 1 à 4 atomes de carbone, un groupe halogénalcoxy contenant de 1 à 3 atomes de carbone et de 1 à 5 atomes d'halogène identiques ou différents, un groupe alkylthio contenant de 1 à 4 atomes de carbone ou représente un groupe halogénalkylthio contenant de 1 à 3 atomes de carbone et de 1 à 5 atomes d'halogène identiques ou différents,
X⁴ représente un atome d'hydrogène, un groupe alkyle à chaîne droite ou ramifiée contenant de 1 à 4 atomes de carbone, un groupe halogénalkyle contenant de 1 à 3 atomes de carbone et de 1 à 5 atomes d'halogène identiques ou différents, un groupe alcoxy contenant de 1 à 4 atomes de carbone, un groupe halogénalcoxy contenant de 1 à 3 atomes de carbone et de 1 à 5 atomes d'halogène identiques ou différents, un groupe alkylthio contenant de 1 à 4 atomes de carbone ou représente un groupe halogénalkylthio contenant de 1 à 3 atomes de carbone et de 1 à 5 atomes d'halogène identiques ou différents,
et
Z représente un atome d'hydrogène ou les radicaux répondant aux formules 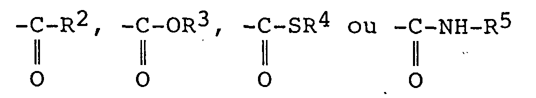 dans lesquelles
R² représente un groupe alkyle à chaîne droite ou ramifiée contenant de 1 à 6 atomes de carbone, un groupe halogénalkyle contenant de 1 à 6 atomes de carbone et de 1 à 7 atomes d'halogène identiques ou différents, un groupe alcényle contenant de 2 à 6 atomes de carbone, un groupe halogénalcényle contenant de 2 à 6 atomes de carbone et de 1 à 7 atomes d'halogène identiques ou différents, un groupe alcoxyalkyle contenant de 1 à 6 atomes de carbone dans la fraction alkyle et de 1 à 6 atomes de carbone dans la fraction alcoxy, un groupe alcoxyalcoxyalkyle contenant de 1 à 6 atomes de carbone dans la fraction alkyle et de 1 à 6 atomes de carbone dans chaque fraction alcoxy, un groupe alkylthioalkyle contenant de 1 à 6 atomes de carbone dans la fraction alkyle et de 1 à 6 atomes de carbone dans la fraction alkylthio, un groupe alkylcarbonyloxyalkyle contenant de 1 à 6 atomes de carbone dans la fraction alkyle et de 1 à 6 atomes de carbone dans la fraction oxyalkyle, un groupe alcénylcarbonyloxyalkyle contenant de 2 à 6 atomes de carbone dans la fraction alcényle et de 1 à 6 atomes de carbone dans la fraction oxyalkyle, ou représente un groupe cycloalkyle contenant de 3 à 8 atomes de carbone et portant le cas échéant de 1 à 3 substituants identiques ou différents halogéno et/ou alkyle contenant de 1 à 4 atomes de carbone, ou
représente un groupe phényle qui peut porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 5 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents, ou
représente un groupe phénylalkyle contenant de 1 à 4 atomes de carbone dans la fraction alkyle, la fraction phényle pouvant porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 5 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents, ou
représente un groupe phénoxyalkyle contenant de 1 à 4 atomes de carbone dans la fraction alkyle, la fraction phényle pouvant porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 3 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents,
R³ représente un groupe alkyle à chaîne droite ou ramifiée contenant de 1 à 6 atomes de carbone, un groupe halogénalkyle contenant de 1 à 6 atomes de carbone et de 1 à 7 atomes d'halogène identiques ou différents, un groupe alcényle contenant de 2 à 6 atomes de carbone, un groupe halogénalcényle contenant de 2 à 6 atomes de carbone et de 1 à 7 atomes d'halogène identiques ou différents, un groupe alcoxyalkyle contenant de 1 à 6 atomes de carbone dans la fraction alkyle et de 1 à 6 atomes de carbone dans la fraction alcoxy, un groupe alcoxyalcoxyalkyle contenant de 1 à 6 atomes de carbone dans la fraction alkyle et de 1 à 6 atomes de carbone dans chaque fraction alcoxy, un groupe alkylthioalkyle contenant de 1 à 6 atomes de carbone dans la fraction alkyle et de 1 à 6 atomes de carbone dans la fraction alkylthio, un groupe alkylcarbonyloxyalkyle contenant de 1 à 6 atomes de carbone dans la fraction alkyle et de 1 à 6 atomes de carbone dans la fraction oxyalkyle, un groupe alcénylcarbonyloxyalkyle contenant de 2 à 6 atomes de carbone dans la fraction alcényle et de 1 à 6 atomes de carbone dans la fraction oxyalkyle, ou représente un groupe cycloalkyle contenant de 3 à 8 atomes de carbone et portant le cas échéant de 1 à 3 substituants identiques ou différents halogéno et/ou alkyle contenant de 1 à 4 atomes de carbone, ou
représente un groupe phényle qui peut porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 5 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents, ou
représente un groupe phénylalkyle contenant de 1 à 4 atomes de carbone dans la fraction alkyle, la fraction phényle pouvant porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 5 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents, ou
représente un groupe phénoxyalkyle contenant de 1 à 4 atomes de carbone dans la fraction alkyle, la fraction phényle pouvant porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 3 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents,
R⁴ représente un groupe alkyle à chaîne droite ou ramifiée contenant de 1 à 6 atomes de carbone, un groupe halogénalkyle contenant de 1 à 6 atomes de carbone et de 1 à 7 atomes d'halogène identiques ou différents, un groupe alcényle contenant de 2 à 6 atomes de carbone, un groupe halogénalcényle contenant de 2 à 6 atomes de carbone et de 1 à 7 atomes d'halogène identiques ou différents, un groupe alcoxyalkyle contenant de 1 à 6 atomes de carbone dans la fraction alkyle et de 1 à 6 atomes de carbone dans la fraction alcoxy, un groupe alcoxyalcoxyalkyle contenant de 1 à 6 atomes de carbone dans la fraction alkyle et de 1 à 6 atomes de carbone dans chaque fraction alcoxy, un groupe alkylthioalkyle contenant de 1 à 6 atomes de carbone dans la fraction alkyle et de 1 à 6 atomes de carbone dans la fraction alkylthio, un groupe alkylcarbonyloxyalkyle contenant de 1 à 6 atomes de carbone dans la fraction alkyle et de 1 à 6 atomes de carbone dans la fraction oxyalkyle, un groupe alcénylcarbonyloxyalkyle contenant de 2 à 6 atomes de carbone dans la fraction alcényle et de 1 à 6 atomes de carbone dans la fraction oxyalkyle, ou représente un groupe cycloalkyle contenant de 3 à 8 atomes de carbone et portant le cas échéant de 1 à 3 substituants identiques ou différents halogéno et/ou alkyle contenant de 1 à 4 atomes de carbone, ou
représente un groupe phényle qui peut porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 5 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents, ou
représente un groupe phénylalkyle contenant de 1 à 4 atomes de carbone dans la fraction alkyle, la fraction phényle pouvant porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 5 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents, ou
représente un groupe phénoxyalkyle contenant de 1 à 4 atomes de carbone dans la fraction alkyle, la fraction phényle pouvant porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 3 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents, et
R⁵ représente un groupe alkyle à chaîne droite ou ramifiée contenant de 1 à 6 atomes de carbone, un groupe halogénalkyle contenant de 1 à 6 atomes de carbone et de 1 à 7 atomes d'halogène identiques ou différents, un groupe alcényle contenant de 2 à 6 atomes de carbone, un groupe halogénalcényle contenant de 2 à 6 atomes de carbone et de 1 à 7 atomes d'halogène identiques ou différents, un groupe alcoxyalkyle contenant de 1 à 6 atomes de carbone dans la fraction alkyle et de 1 à 6 atomes de carbone dans la fraction alcoxy, un groupe alcoxyalcoxyalkyle contenant de 1 à 6 atomes de carbone dans la fraction alkyle et de 1 à 6 atomes de carbone dans chaque fraction alcoxy, un groupe alkylthioalkyle contenant de 1 à 6 atomes de carbone dans la fraction alkyle et de 1 à 6 atomes de carbone dans la fraction alkylthio, un groupe alkylcarbonyloxyalkyle contenant de 1 à 6 atomes de carbone dans la fraction alkyle et de 1 à 6 atomes de carbone dans la fraction oxyalkyle, un groupe alcénylcarbonyloxyalkyle contenant de 2 à 6 atomes de carbone dans la fraction alcényle et de 1 à 6 atomes de carbone dans la fraction oxyalkyle, ou représente un groupe cycloalkyle contenant de 3 à 8 atomes de carbone et portant le cas échéant de 1 à 3 substituants identiques ou différents halogéno et/ou alkyle contenant de 1 à 4 atomes de carbone, ou
représente un groupe phényle qui peut porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 5 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents, ou
représente un groupe phénylalkyle contenant de 1 à 4 atomes de carbone dans la fraction alkyle, la fraction phényle pouvant porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 5 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents, ou
représente un groupe phénoxyalkyle contenant de 1 à 4 atomes de carbone dans la fraction alkyle, la fraction phényle pouvant porter de 1 à 3 substituants identiques ou différents halogéno, alkyle contenant de 1 à 4 atomes de carbone, halogénalkyle contenant de 1 à 3 atomes de carbone et de 1 à 5 atomes d'halogène identiques ou différents, alcoxy contenant de 1 à 4 atomes de carbone, halogénalcoxy contenant de 1 à 3 atomes de carbone et de 1 à 3 atomes d'halogène identiques ou différents, alkylthio contenant de 1 à 4 atomes de carbone et/ou halogénalkylthio contenant de 1 à 3 atomes de carbone et de 1 à 5 atomes d'halogène identiques ou différents,

3. Procédé pour la préparation d'anilides d'acides cycloalkylcarboxyliques répondant à la formule 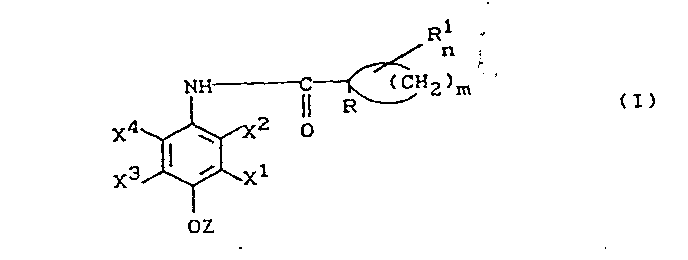 dans laquelle
R représente un atome d'halogène ou un groupe halogénalkyle,
R¹ représente un atome d'halogène, un groupe alkyle ou un groupe halogénalkyle,
m représente des nombres entiers de 2 à 7,
n représente les nombres 0, 1, 2 ou 3,
X¹, X², X³ et X⁴ représentent, indépendamment l'un de l'autre un atome d'hydrogène, un atome d'halogène, un groupe alkyle, un groupe halogénalkyle, un groupe alcoxy, un groupe halogénalcoxy, un groupe alkylthio ou un groupe halogénalkylthio, et
Z représente un atome d'hydrogène ou les radicaux répondant aux formules 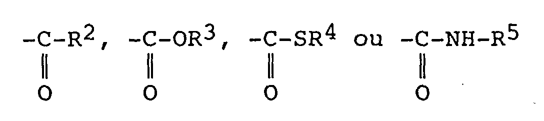 dans lesquelles
R² représente un groupe alkyle, un groupe halogénalkyle, un groupe alcényle, un groupe halogénalcényle, un groupe alcoxyalkyle, un groupe polyalcoxyalkyle, un groupe alkylthioalkyle, un groupe alkylcarbonyloxyalkyle, un groupe alcénylcarbonyloxyalkyle, un groupe cycloalkyle le cas échéant substitué, un groupe phényle le cas échéant substitué, représente un groupe phénylalkyle le cas échéant substitué ou représente un groupe phénoxyalkyle le cas échéant substitué,
R³ représente un groupe alkyle, un groupe halogénalkyle, un groupe alcényle, un groupe halogénalcényle, un groupe alcoxyalkyle, un groupe polyalcoxyalkyle, un groupe alkylthioalkyle, un groupe alkylcarbonyloxyalkyle, un groupe alcénylcarbonyloxyalkyle, un groupe cycloalkyle le cas échéant substitué, un groupe phényle le cas échéant substitué, représente un groupe phénylalkyle le cas échéant substitué ou représente un groupe phénoxyalkyle le cas échéant substitué,
R⁴ représente un groupe alkyle, un groupe halogénalkyle, un groupe alcényle, un groupe halogénalcényle, un groupe alcoxyalkyle, un groupe polyalcoxyalkyle, un groupe alkylthioalkyle, un groupe alkylcarbonyloxyalkyle, un groupe alcénylcarbonyloxyalkyle, un groupe cycloalkyle le cas échéant substitué, un groupe phényle le cas échéant substitué, représente un groupe phénylalkyle le cas échéant substitué ou représente un groupe phénoxyalkyle le cas échéant substitué, et
R⁵ représente un groupe alkyle, un groupe halogénalkyle, un groupe alcényle, un groupe halogénalcényle, un groupe alcoxyalkyle, un groupe polyalcoxyalkyle, un groupe alkylthioalkyle, un groupe alkylcarbonyloxyalkyle, un groupe alcénylcarbonyloxyalkyle, un groupe cycloalkyle le cas échéant substitué, un groupe phényle le cas échéant substitué, représente un groupe phénylalkyle le cas échéant substitué ou représente un groupe phénoxyalkyle le cas échéant substitué,
caractérisés en ce qu'on fait réagir des aminophénols répondant à la formule 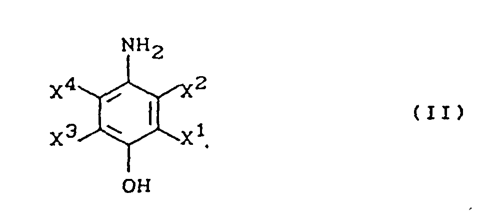 dans laquelle
X¹, X², X³ et X⁴ ont les significations indiquées ci-dessus,
avec des dérivés d'acides cycloalkylcarboxyliques répondant à la formule 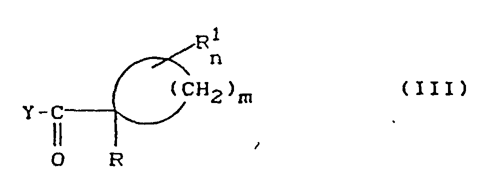 dans laquelle
R, R¹, m et n ont les significations indiquées ci-dessus et
Y représente un groupe qui s'éloigne,
le cas échéant en présence d'un agent neutralisant les acides et le cas échéant en présence d'un diluant et, le cas échéant, on fait réagir les anilides d'acides cycloalkylcarboxyliques que l'on obtient en l'occurrence répondant à la formule 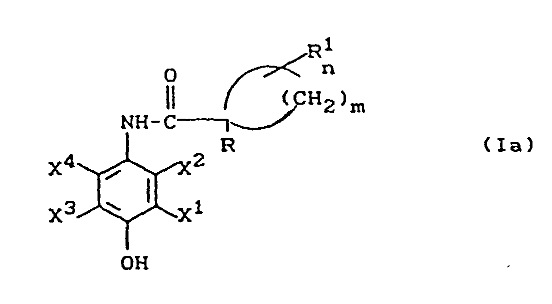 dans laquelle
R, R¹, m, n, X¹, X², X³ et X⁴ ont les significations indiquées ci-dessus,
soit
a) avec des halogénures d'acides carboxyliques répondant à la formule 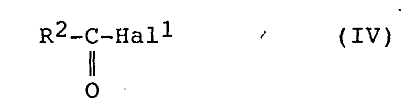 dans laquelle
R² a la signification indiquée ci-dessus et
Hal¹ représente un atome d'halogène,
le cas échéant en présence d'un agent neutralisant les acides et le cas échéant en présence d'un diluant
soit
b) avec des esters halogénoformiques répondant à la formule 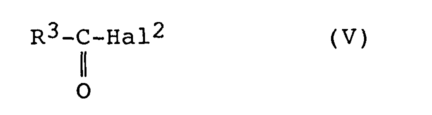 dans laquelle
R³ a la signification indiquée ci-dessus et
Hal² représente un atome d'halogène,
le cas échéant en présence d'un agent neutralisant les acides et le cas échéant en présence d'un diluant,
soit
c) avec des thioesters halogénoformiques répondant à la formule 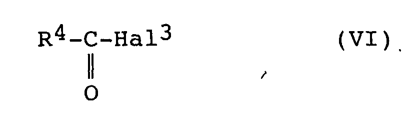 dans laquelle
R⁴ a la signification indiquée ci-dessus et
Hal³ représente un atome d'halogène,
le cas échéant en présence d'un agent neutralisant les acides et le cas échéant en présence d'un diluant,
soit
d) avec des isocyanates répondant à la formule
R⁵-NCO (VII)
dans laquelle
R⁵ a la signification indiquée ci-dessus,
le cas échéant en présence d'un diluant et le cas échéant en présence d'un catalyseur.

4. Agents de lutte contre les parasites caractérisés par une teneur en au moins un anilide d'acide cycloalkylcarboxylique répondant à la formule (I) selon la revendication 1.

5. Utilisation d'anilides d'acides cycloalkylcarboxyliques répondant à la formule (I) selon la revendication 1 pour lutter contre les parasites.

6. Procédé pour lutter contre des parasites, caractérisé en ce qu'on répand des anilides d'acides cycloalkylcarboxyliques répondant à la formule (I) selon la revendication 1 sur les parasites et/ou sur leur biotope.

7. Procédé de préparation d'agents de lutte contre les parasites caractérisé en ce qu'on mélange des anilides d'acides cycloalkylcarboxyliques répondant à la formule (I) selon la revendication 1 avec des diluants et/ou des substances tensioactives.
